# EUROPEAN PATENT APPLICATION

(11) **EP 4 663 199 A1**
(43) Date of publication of application: **17.12.2025**
(21) Application number: 24181745.1
(22) Date of filing: 12.06.2024
(51) Int. Cl.: A61K 38/17, A61K 39/395, C07K 14/33, C07K 14/775, C12N 15/88

(54) **FUSION POLYPEPTIDE AND LIPONANOPARTICLE COMPRISING THE SAME**

(71) Applicant: Strike Pharma AB, 756 41 Uppsala (SE); Circular Biotech AB, 90741 Umeå (SE)
(72) Inventor: MANGSBO, Sara, 75641 Uppsala (SE); NILSSON, Jonas, 90741 Umeå (SE); LIDSTRÖM, Tommy, 90741 Umeå (SE)
(74) Representative: Dehns

(57) **Abstract**

The present disclosure relates to fusion polypeptide comprising a tag moiety and a polypeptide comprising an apolipoprotein or a fragment thereof, for example for. delivery of a loaded decorated liponanoparticle attached via the apoliprotein or a fragment thereof to a cell of interest, wherein said loaded decorated liponanopaticle comprises a payload. Also disclosed are loaded decorated liponanoparticles comprising said fusion polypeptide and a complex comprising a bispecific bindning molecule and the loaded decorated liponanoparticle. Medical uses of the fusion polypeptide, loaded decorated liponanoparticle and complex as disclosed herein are also provided.

## Description

### Technical field

The present disclosure relates to fusion polypeptide comprising a tag moiety and a polypeptide comprising an apolipoprotein or a fragment thereof. Said fusion polypeptide may for be useful for the targeted delivery of a loaded decorated liponanoparticle (LNP) to a cell, tissue or organ of interest, wherein said loaded decorated liponanopaticle comprises a payload. Also disclosed are loaded decorated liponanoparticles comprising said fusion polypeptide. In addition the present disclosure relates to a complex comprising a bispecific bindning molecule and the loaded decorated liponanoparticle, which comprises at least one first specific binding molecule with affinity for an antigen of interest and at least one second specific binding molecule with affinity for said tag moiety, wherein said loaded decorated liponanoparticle is non-covalently bound to said second specific binding molecule via said tag moiety. Medical uses of the fusion polypeptide, loaded decorated liponanoparticle and complex as disclosed herein are also provided.

### Background

The delivery of payload, such as oligonucleotides, to cells can be achieved via a fine-tuned balance of liponanoparticle (LNP) composition in which said payload, such as for example mRNA, is incorporated. The tuning of the amount of lipoproteins and payload will determine the net charge of the formed nanoparticle which can change the organ to which the nanoparticle it directed to, and thereby the cellular uptake based on that targeting. However, the charge-based targeting is still a crude method for targeted delivery and alternative methods are required to facilitate directed methods of oligonucleotide-based therapies.

Methods can include the use of incorporation of certain proteins for natural change of biodistribution patterns based on these protein interactions *in vivo.* For example antibodies may be used for targeted delivery and it is possible to build in antibodies into the liponanoparticle structures, but introducing proteins into the liponanoparticle itself requires modification to enable protein-lipid interaction and stability of the formed LNP; which is hard to standardize between antibody variants. While the antibodies built into liponanoparticles may useful for targeted of the liponanoparticles, each specific target requires the design of a new liponanoparticle with an antibody incorporated therein, making this approach technically challenging and inflexible. Thus, there is a need in the field to provide a system for a better and simple targeting of liponanoparticles for delivering payloads to desired organs, tissues and/or cells. In particular, it is desirable that such system retains a structural flexibility in said payload delivery utilizing liponanoparticles, allowing for easy target modification such that payload may be delivered by targeted delivery to various targets needed. The present invention addresses this need.

### Disclosure of the invention

It is an object of the present disclosure to meet this need through the provision of a fusion polypeptide comprising a tag moiety and a polypeptide comprising an apolipoprotein or a fragment thereof. Said fusion polypeptide is designed to have the ability to be coupled to the surface layer of a liponanoparticle (LNP) therein resulting in a decorated liponanoparticle. When said fusion polypeptide is coupled to the surface layer of a liponanoparticle, it provides a non-covalent and flexible binding link between said decorated LNP and any molecule with binding affinity for the comprised tag moiety sequence.

It is another object of the disclosure to provide a payload delivery vessel in the form of a loaded decorated liponanoparticle (LNP) comprising a core which comprises a payload, wherein said loaded decorated liponanoparticle further comprises components allowing the LNP to be flexibly and non-covalently bound to other molecules or cells.

It is a further object of the disclosure to provide a complex comprising said payload delivery vessel, in the form of a loaded decorated LNP, wherein said loaded decorated LNP, and the payload it comprises, can be directed to specific molecular, cellular, tissue and/or organ targets. Said complex provides for a flexible system, wherein both the target and the payload can be tailored to the need at hand, while the components of the system are readily and efficiently scalable.

Yet another object of the disclosure is to enable the use of said fusion polypeptide, loaded decorated liponanoparticle and complex, as well as compositions and kits comprising the aforementioned components.

Yet another further object of the disclosure is to provide methods of production of the aforementioned components, as well as applications in therapy.

These and other objects which are evident to the skilled person from the present disclosure are met by different aspects of the invention as claimed in the appended claims and as generally disclosed herein.

The present inventors have surprisingly found these objects are met by a system of providing lipid-interacting proteins with a tag moiety that has high affinity for a binding molecule. The inventors thereby improve liponanoparticle based therapeutics by adding a means for targeted delivery to specific organs, tissues and/or cell types. This approach allows for standardization of the production of said improved liponanoparticles with coupled fusion polypeptides, which fusion polypeptides comprise a tag moiety, and allow for targeting of the liponanoparticle to a desired organ, tissue and/or cell. The targeting is based on the use of a bispecific binding molecule, which is designed to capture the tag moiety with a high affinity (in the subnanomolar range) interaction as well as to target the desired organ, tissue and/or cell, based on affinity therefor.

The present invention is advantageous as it can be adapted for use in all existing and future liponanoparticle therapeutics without the need to chemically, or by other means, adapt or modify liponanoparticle assembly. This is envisioned to broaden the use of payload-based therapies, such as for example, but not limited to, nucleic acid payload based-therapies, and allow for standardized method development of liponanoparticles without a need for adapting said method for each new protein to be coupled to the liponanoparticle or incorporated into the liponanoparticle surface, in contrast to methods and systems known in the art.

Thus, in a first aspect of the present disclosure, there is provided a fusion polypeptide comprising
i) a tag moiety comprising or consisting of the amino acid sequence FIGITELX₈X₉ (SEQ ID NO:1), wherein X₈ is selected from K, L, and H; and X₉ is K or absent; and
ii) a polypeptide comprising an apolipoprotein or a fragment thereof,
said tag moiety is arranged N-terminally of said apolipoprotein or fragment thereof and wherein said fusion polypeptide optionally comprises a linker sequence arranged between said tag moiety and said apolipoprotein or fragment thereof.

Said tag moiety comprises at least one epitope, such as one epitope, for binding by a binding molecule specific therefore. It is to be understood that said at least one epitope is located within the amino acid sequence FIGITELX₈X₉ (SEQ ID NO:1).

In one embodiment, said fusion polypeptide comprises
i) a tag moiety comprising or consisting of, such as consisting of, the amino acid sequence FIGITELX₈X₉ (SEQ ID NO:1), wherein X₈ is selected from K, L, and H; and X₉ is K or absent; and
ii) a polypeptide comprising or consisting of, such as consisting of, an apolipoprotein or a fragment thereof, comprising or consisting of, such as consisting of, said tag moiety is arranged N-terminally of said apolipoprotein or fragment thereof and wherein said fusion polypeptide optionally comprises a linker sequence arranged between said tag moiety and said apolipoprotein or fragment thereof.

In one embodiment, there is provided a fusion polypeptide wherein said tag moiety consists of the amino acid sequence FIGITELX₈X₉ (SEQ ID NO:1), wherein X₈ is selected from K, L, and H; and X₉ is K or absent. Thus, the tag moiety is 8 or 9 amino acids long. In the specific embodiment, wherein said tag moiety consists of the amino acid sequence FIGITELX₈X₉ (SEQ ID NO:1), it is understood that said epitope is located within the amino acid sequence FIGITELX₈X₉. In this case, said tag moiety comprises at least one epitope, such as one epitope, for binding by a binding molecule specific therefore.

It is to be understood that the wording "located within the amino acid sequence FIGITELX₈X₉ (SEQ ID NO:1) in the context of said tag moiety comprising or consisting of SEQ ID NO:1 is not to be interpreted as located completely or partially external to said sequence. It is due to the presence of said epitope within SEQ ID NO:1 that a second specific binding molecule as defined below is able to specifically bind to said tag moiety.

In embodiments, wherein said tag moiety comprises the amino acid sequence FIGITELX₈X₉ (SEQ ID NO:1), the tag moiety may comprise additional epitopes for binding a binding molecule specific therefore.

In one embodiment, X₈ is selected from K and L.

In one embodiment, X₈ is selected from L and H.

In one embodiment, X₈ is selected from K and H.

In one embodiment, X₈ is K. In yet another embodiment, X₈ is L. In still another embodiment, X₈ is H.

In one embodiment, X₉ is K. In another embodiment, X₉ is absent.

Said alternative embodiments of the amino acids in positions X₈ and X₉ may be freely combined in any subgroup. For example, in one embodiment, the tag moiety comprises or consists of an amino acid sequence selected from the group consisting of SEQ ID NO:2-7.

In one embodiment, the amino acid sequence is selected from the group consisting of SEQ ID NO:2-4. In another embodiment, the amino acid sequence is selected from the group consisting of SEQ ID NO:5-7.

In one embodiment, the amino acid sequence is selected from the group consisting of SEQ ID NO:2 and 3, or the group consisting of SEQ ID NO: 2 and 4, or the group consisting of SEQ ID NO:3 and 4.

In one embodiment, the tag moiety comprises or consists of the amino acid sequence is SEQ ID NO:2. In one embodiment, the tag moiety comprises or consists of the amino acid sequence is SEQ ID NO:3. In one embodiment, the tag moiety comprises or consists of the amino acid sequence is SEQ ID NO:4.

As explained above, the tag moiety as disclosed herein is useful as a binding target, in other words epitope, for the binding molecule specific therefor. In embodiments, wherein the tag moiety consists of 8 or 9 amino acid residues as specified herein, the tag moiety is considered a minimal binding target for said binding molecule specific therefor. Without being bound by theory, a minimal target may minimize the risk for unspecific binding as well as not posing size constrains on the polypeptide comprising an apolipoprotein or a fragment thereof. This property makes it useful e.g., in the context of a drug platform such as that described in WO2020/104690 and WO2021/239968, insofar this platform incorporates a binding molecule specific for the tag moiety in a bispecific conjugate molecule. It may be advantageous that the tag moiety does not comprise any B-cell epitopes or at least no natural B-cell epitope (for example human B-cell epitopes) may be non-immunogenic, in order to avoid the generation of an immune response to the tag moiety when it is administered to a subject. By a "natural B-cell epitope" is meant an epitope against which a subject, for example a human (in particular a patient to whom the conjugate is to be administered), has no circulating antibodies prior to administration of the conjugate.

As the skilled person understands, the construction of a fusion polypeptide often, but not always, involves the use of linkers between functional moieties to be fused. The skilled person is aware of different kinds of linkers with different properties, such as flexible amino acid linkers, rigid amino acid linkers and cleavable amino acid linkers. Linkers have been used to for example increase stability or improve folding of fusion polypeptides, to increase expression, improve biological activity, enable targeting and alter pharmacokinetics of fusion polypeptides. Thus, the fusion polypeptide as disclosed herein may comprise a linker sequence in some embodiments. In such cases the fusions polypeptide may schematically be represented as N'-[tag moiety]-[linker]-[apolipoprotein or fragment thereof]-C'. However, as shown in the appended examples, the presence of a linker is optional and fusions polypeptide without a linker schematically be represented as N'-[tag moiety]- [apolipoprotein or fragment thereof]- C', wherein the amino acid at the C-terminus of the tag moiety is fused the amino acid at the N-terminus of the apolipoprotein or fragment thereof. In one embodiment, said C terminus of said tag moiety is covalently bound to the N terminus of said apolipoprotein or a fragment thereof. In one embodiment, said fusion polypeptide comprises a linker sequence arranged between said tag moiety and said apolipoprotein or fragment thereof.

In one embodiment, said linker is a flexible linker, a rigid linker or a cleavable linker, such as a flexible linker.

Flexible linkers are often used in the art when the joined domains require a certain degree of movement or interaction, and may be useful in some embodiments of the fusion polypeptide as described herein. Such linkers are generally composed of small, non-polar (for example G) or polar (for example S or T) amino acids. Some flexible linkers primarily consist of stretches of G and S residues, for example (GGGGS)p and (SSSSG)p (wherein GGGGS corresponds to SEQ ID NO:9 and SSSSG corresponds to SEQ ID NO:414. Adjusting the copy number "p" allows for optimization of the linker in order to achieve appropriate separation between the functional moieties or to maintain necessary inter-moiety interaction. Apart from G and S linkers, other flexible linkers are known in the art, such as G and S linkers containing additional amino acid residues, such as T, A, K and E, to maintain flexibility as well as polar amino acid residues to improve solubility. In one embodiment, said flexible linker is selected from the group consisting of GGGGS (SEQ ID NO:9), GGGGSGGGGS (SEQ ID NO:10) and GGGGSGGGGSGGGGS (SEQ ID NO:11).

Additional non-limiting examples of linkers include GGGGSLVPRGSGGGGS (SEQ ID NO:415), (GS)₃ (SEQ ID NO:416), (GS)₄ (SEQ ID NO:417), (GS)₈ (SEQ ID NO:418), GGSGGHMGSGG (SEQ ID NO:419), GGSGGSGGSGG (SEQ ID NO:420), GGSGG (SEQ ID NO:421), GGSGGGGG (SEQ ID NO:422), GGGSEGGGSEGGGSEGGG (SEQ ID NO:423), AAGAATAA (SEQ ID NO:424), GGGGG (SEQ ID NO:425), GGSSG (SEQ ID NO:426), GSGGGTGGGSG (SEQ ID NO:427), GSGGGTGGGSG (SEQ ID NO:428), GT, GGS, GSGSGSGSGGSG (SEQ ID NO:429), GSGGSGGSGGSGGS (SEQ ID NO:430) and GSGGSGSGGSGGSG (SEQ ID NO:431). The skilled person is aware of other suitable linkers.

In one embodiment, said linker is a flexible linker comprising glycine (G), serine (S) and/or threonine (T) residues. In one embodiment, said linker has a general formula selected from (GnSm)p and (SmGn)p, wherein, independently, n = 1-7, m = 0-7, n + m ≤ 8 and p = 1-7. In one embodiment, n = 1-5. In one embodiment, m = 0-5. In one embodiment, p = 1-5. In a more specific embodiment, n = 4, m = 1 and p = 1-4. In one embodiment, said linker is selected from the group consisting of S₄G (SEQ ID NO:414), (S₄G)₃ (SEQ ID NO:432) and (S₄G)₄(SEQ ID NO:433). In one embodiment, said linker is selected from the group consisting of G₄S and (G₄S)₃. In one particular embodiment, said linker is G₄S and in another embodiment said linker is (G₄S)₃. In one embodiment, said linker has the general formula (GₑT_{f})_{q}, wherein, independently, e = 1-3, f = 1-3, and q = 1-4. In one embodiment, q = 1-3. In a more specific embodiment, e = 1, f = 1 and q = 1-3. In one embodiment, said linker is selected from the group consisting of GT, (GT)₂ (SEQ ID NO:434) and (GT)₃ (SEQ ID NO:435). In one embodiment, said linker is GT. In one embodiment, the linker is EF. In one embodiment, said linker is a rigid linker. Rigid linkers may be particularly useful in distancing the Tag moiety from the polypeptide comprising an apolipoprotein or a fragment thereof which could enhance binding to the bispecific antibody as the distancing could render full accessibility to a certain structure. In one particular embodiment, said rigid linker is selected from the group consisting of EAAAK (SEQ ID NO:12), EAAAKEAAAK (SEQ ID NO:13) and EAAAKEAAAKEAAAK (SEQ ID NO:14).

In one embodiment, said linker is a cleavable linker. Cleavable linkers may be particularly useful in enhancing endosomal escape following targeted uptake of the payload of interest and/or to off-load a payload in a certain cell based on a certain pH or a certain enzymatic activity present in a certain intracellular structure of a cell. In one particular embodiment, said linker is a cleavable linker selected from the group consisting of GFLG (SEQ ID NO:328), PLGLWA (SEQ ID NO:329) and AGNRVRRSVG (SEQ ID NO:330).

The skilled person is aware of other suitable rigid linkers and cleavable linkers which may be used in the context of the fusion polypeptide as disclosed herein.

In some embodiments, said linker may comprise a processing element, for example a proceeding element which is proteasomal cleavage site. Examples of such processing elements are AAA and AAY.

In one embodiment, said fusion polypeptide does not comprise a linker.

The term "apolipoprotein" as used herein refers to a protein that together with lipids forms lipoproteins, i.e. assemblies of lipids and proteins. As used herein, the term encompasses wild-type apolipoproteins, such as in particular human wild-type apolipoproteins, as well as biologically active fragments thereof, biologically active variants of apolipoproteins or biologically active fragments thereof, including biologically active mutants (such as naturally occurring mutant or non-naturally occurring mutant) apolipoproteins or biologically active fragments thereof. The term "apoliproptein" is to be interpreted as encompassing native apolipoproteins, fragments thereof as well as apolipoprotein mimetics, which are molecules with a function resembling that of apolipoproteins and can encapsulate liponanoparticles with a shorter peptide than the full length apolipoprotein. Liponanoparticles comprising apolipoprotein mimetics are considered to have low toxic effects. A "apolipoprotein fragment" in the context of the present invention is a fragment that at least substantially retains the lipid binding property of the full length apolipoprotein, such as an apolipoprotein fragment which subtantially comprises the lipid binding domain, such as comprises the complete lipid binding domain, of the apolipoprotein. Apolipoproteins typically function to transport lipids and fat-soluble substances in the blood. Apolipoproteins are described and include but are not limited to ApoA1, ApoA2, ApoA4, ApoA5, ApoB48, ApoC1, ApoC2, ApoC3, ApoC4, ApoD, ApoE (including isoforms ApoE2, ApoE3 and ApoE4), ApoH and ApoM.

The term "fragment" as used herein in reference to a polypeptide or protein refers to a portion of the polypeptide or protein. Such fragment may be an N- and/or C-terminally truncated form of said polypeptide or protein. Examples of fragment of apolipoprotein according to the present disclosure include but are not limited to N-terminal truncations of the wildtype apolipoprotein. Such truncations may be fused to the tag moiety as defined herein. In the context of the present fusion polypeptide, the apolipoprotein or fragment thereof does not comprise a N-terminal signal peptide.

In one embodiment of the fusion polypeptide as defined herein, the apolipoprotein or fragment thereof is selected from the group consisting of ApoA1 (SEQ ID NO:402), ApoA2 (SEQ ID NO:403), ApoA4 (SEQ ID NO:404), ApoA5 (SEQ ID NO:405), ApoB48 (SEQ ID NO:406), ApoC1 (SEQ ID NO:407), ApoC2 (SEQ ID NO:408), ApoC3 (SEQ ID NO:409), ApoC4 (SEQ ID NO:410), ApoD (SEQ ID NO:411), ApoE2 (SEQ ID NO:283), ApoE3 (SEQ ID NO:284), ApoE4 (SEQ ID NO:285), ApoH (SEQ ID NO:412), ApoM (SEQ ID NO:413) and isoforms thereof.

In one embodiment, said the apolipoprotein or fragment thereof is selected from the group consisting of ApoA, ApoC, ApoE and any isoforms thereof, such as selected form the group consisting of ApoA1 (SEQ ID NO:402), ApoA2 (SEQ ID NO:403), ApoA4 (SEQ ID NO:404), ApoA5 (SEQ ID NO:405), ApoC1 (SEQ ID NO:407), ApoC2 (SEQ ID NO:408), ApoC3 (SEQ ID NO:409), ApoC4 (SEQ ID NO:410), ApoE2 (SEQ ID NO:283), ApoE3 (SEQ ID NO:284), ApoE4 (SEQ ID NO:285). In one embodiment, wherein said apolipoprotein or fragment thereof is selected from the ApoE and isoforms thereof.

In one embodiment, said apolipoprotein is selected from the group consisting of ApoE isoform 2 (SEQ ID NO:283), ApoE isoform 3 (SEQ ID NO:284) and ApoE isoform 4 (SEQ ID NO:285) and fragments thereof, such as wherein said apolipoprotein is ApoE isoform 2 (SEQ ID NO:283) or a fragment thereof.

In one embodiment said fragment is a truncated form of an apolipoprotein, such as a truncated from of an isoform of ApoE. In one embodiment, said fragment is a truncated form of ApoE isoform 2 (SEQ ID NO:283), ApoE isoform 3 (SEQ ID NO:284) or ApoE isoform 4 (SEQ ID NO:285). In one particular embodiment, said fragment is a truncated form of ApoE2 (SEQ ID NO:283). In one embodiment, said fragment is a N-terminally and/or a C-terminally truncated form. In one embodiment, said fragment is a C-terminally truncated form. In one embodiment, said fragment is a N-terminally truncated form.

In embodiment there is provided a fusion polypeptide as disclosed herein, wherein said apolipoprotein or fragment thereof comprises a least one mutation, such as one, two, three, four or five or more mutations. Said mutation may be present in order to reduce unwanted affinity of said apolipoprotein or fragment thereof for its *in vivo* targets and thus decrease binding to said targets, such as with the LDL-Receptor (LDL-R) and/or with heparan sulphate proteoglycan. Mutations may also affect the interactions of said apolipoprotein with lipid particles. Without being bound by theory, the present inventors envision that by decreasing the binding to the said apolipoprotein or fragment thereof to said targets, such as LDL-Receptor (LDL-R) and/or with heparan sulphate proteoglycan, the binding competition and resulting potential sterical hindrance, the specific binding to the tag moiety by a binder which has affinity therefore may be more efficient.

Thus, in one embodiment, said at least one mutation, such as two, three, four or five mutations or more, reduce(s ) the affinity of the apolipoprotein or fragment thereof for LDL-R and/or heparan sulphate proteoglycan, such as wherein said at least one mutation, such as two, three, four or five or more mutations, abolish(es) the affinity of the apolipoprotein or fragment thereof for LDL-R and/or heparan sulphate proteoglycan. In one embodiment, said at least one mutation, such as two, three, four or five mutations or more, reduce(s ) the affinity of the apolipoprotein or fragment thereof for LDL-R, such as wherein said at least one mutation, such as two, three, four or five or more mutations, abolish(es) the affinity of the apolipoprotein or fragment thereof for LDL-R. In one embodiment, said at least one mutation, such as two, three, four or five mutations or more, reduce(s ) the affinity of the apolipoprotein or fragment thereof for heparan sulphate proteoglycan , such as wherein said at least one mutation, such as two, three, four or five or more mutations, abolish(es) the affinity of the apolipoprotein or fragment thereof and/or heparan sulphate proteoglycan.

In one particular embodiment, said at least one mutation is located in the heparan sulphate proteoglycan binding domain of said apolipoprotein or fragment thereof, such as wherein said at least one mutation is located in the region as defined by amino acid residues 144-147 as defined in SEQ ID NO:283 or in the corresponding region of a fragment thereof. In one embodiment, said at least one mutation is located in the LDL-R binding domain of said apolipoprotein or fragment thereof, such as wherein said at least one mutation is located in the region as defined by amino acid residues 140-150 as defined in SEQ ID NO:283 or in the corresponding region of a fragment thereof.

In one embodiment said at least one mutation, such as two, three, four or five mutations or more, is/are located in a mutation site region, which region corresponds to the amino acid sequence/residues 132-158 ELRVRLASHLRKLRKRLLRDADDLQKC (SEQ ID NO:387) of ApoE isoform 2 (SEQ ID NO:283) or in the corresponding region of a fragment thereof.

In one particular embodiment, said at least one mutation, such as two, three, four or five mutations, is/are selected from mutations in amino acid positions selected form the group consisting of 136, 140, 143, 144 and 150 as defined in ApoE2 (SEQ ID NO:283) or in the corresponding amino acid positions of a fragment thereof. In one particular embodiment, said at least one mutation, such as two, three, four or five mutations, is/are selected from the group consisting of R136S, H140A, K143A, L144P, and R150A, as defined in ApoE2 (SEQ ID NO:283) or in the corresponding region of a fragment thereof. In one particular embodiment, said at least one mutation, such as two, three, four or five mutations, is/are selected from the group consisting of R136S, H140A, K143A, L144P, and R150A, in amino acid positions 136, 140, 143, 144 and 150 as defined in ApoE2 (SEQ ID NO:283) or in the corresponding amino acid positions of a fragment thereof.

Examples of one to three mutations within LDL-R/Heparan sulphate proteoglycan binding domain ELRVRLASHLRKLRKRLLRDADDLQKC (SEQ ID NO:387) of SEQ ID NO:283 are illustrated by SEQ ID NO: 389, 18 and 391-401 wherein both the LDLR binding domain and the heparan sulphate proteoglycan binding domain have been disrupted.

In one particular embodiment, said at least one mutation is R136S as defined in ApoE2 (SEQ ID NO:283) or in the corresponding region of a fragment thereof. In one embodiment, said fusion polypeptide comprises one, two, three, four or more mutation in addition to said R136S mutation. In one specific embodiment of the fusion polypeptide as disclosed herein, said apolipoprotein or fragment thereof is SEQ ID NO:18 or a fragment thereof.

In one particular embodiment, the fusion polypeptide comprises a mutation in amino acid position 61 as defined in ApoE2 (SEQ ID NO:283) or in the corresponding amino acid position of a fragment thereof. In one particular embodiment, the fusion polypeptide comprises the mutation R61T in said apolipoprotein or fragment thereof, such as comprises the mutation R61T as defined in ApoE2 (SEQ ID NO:283) or in the corresponding region of a fragment thereof. In one particular embodiment, mutation R61T is in amino acid position 61 as defined in ApoE2 (SEQ ID NO:283) or in the corresponding amino acid position of a fragment thereof. ApoE2 comprising mutation R61T is shown in SEQ ID NO:389.

In one specific embodiment of the fusion polypeptide as disclosed herein, said apolipoprotein or fragment thereof is SEQ ID NO:389 or a fragment thereof.

Thus, in one embodiment, said fusion polypeptide comprises one, two or three mutations in the sequence defined by ApoE2 (SEQ ID NO:283) or in the corresponding region of a fragment thereof, selected from mutations in amino acid residue positions selected from the group consisting of: 61; 136; 140; 143; 144; 150; 136 and 140; 136 and 144; 136 and 140 and 144; 136 and 143; 136 and 150; 136 and 143 and 150; and 136 and 61. Thus, in one embodiment, said fusion polypeptide comprises one, two or three mutations in the sequence defined by ApoE2 (SEQ ID NO:283) or in the corresponding region of a fragment thereof, selected from the group consisting of: R61T; R136S; R140A; K143A; L144P; R150A; R136S and H140A; R136S and L144P; R136S and H140A and L144P; R136S and K143A; R136S and R150A; R136S and K143A and R150A; and R136S and R61T. It will be understood, that said fragment comprises the relevant mutations. In one embodiment, said fusion polypeptide as disclosed herein comprises an apolipoprotein or a fragment thereof selected from the group consisting of SEQ ID NO: 389, 18, 391-401 and fragments thereof, such as selected from the group consisting of SEQ ID NO:18 and 395-401 and fragments thereof, such as wherein said fusion polypeptide comprises SEQ ID NO:18 or a fragment thereof. In one embodiment, said apolipoprotein or fragment thereof is selected from the group consisting of SEQ ID NO:18 and 395-401 and fragments thereof, such as wherein said apolipoprotein or fragment thereof is selected from the group consisting of SEQ ID NO:18 and fragments thereof, which fragments comprise the R136S mutation.

It is also envisioned, that parts of the apolipoprotein or of fragments thereof may be deleted, such as deleted to remove the LDL-R and/heparan sulphate proteoglycan binding activity thereof. Thus, in one embodiment there is provided a fusion polypeptide as defined herein, wherein said apolipoprotein or fragment thereof does not comprise a heparan sulphate proteoglycan binding domain or comprises a heparan sulphate proteoglycan binding domain with reduced affinity for heparan sulphate proteoglycan compared to the wildtype domain, such as wherein said apolipoprotein or fragment thereof does not comprise a heparan sulphate proteoglycan binding domain. In one embodiment, said apolipoprotein or fragment thereof does not comprise the amino acid residues 144-147 as defined in SEQ ID NO:283. In one embodiment there is provided a fusion polypeptide as defined herein, wherein said apolipoprotein or fragment thereof does not comprise an LDL-R binding domain or comprises a LDL-R binding domain with reduced affinity for heparan sulphate proteoglycan compared to the wildtype domain, such as wherein said apolipoprotein or fragment thereof or does not comprise a LDL-R binding domain. In one embodiment, said apolipoprotein or fragment thereof does not comprise amino acid residues 140-150 as defined in SEQ ID NO:283.

As generally discussed above, in some embodiments, there is provided a fusion polypeptide wherein the apolipoprotein is a N-terminally truncated fragment and is used to the C-terminal part of the tag moiety as defined herein. The truncation may for example be of in the range of 1 to 201 amino acid residues from the N-terminal of the apolipoprotein, such as ApoE or any isoform thereof, such as ApoE2 as defined herein by SEQ ID NO:283 or ApoE comprising the mutation R136S as defined herein by SEQ ID NO:18. In particular embodiments, said truncation may be of 1-11 amino acid residues from the N-terminal, of 42-55 amino acid residues from the N-terminal or 172-201 amino acid residues from the N-terminal of ApoE2 as defined herein by SEQ ID NO:283 or by any one of SEQ ID NO:389, 18, 391-401. Thus, in one embodiment, there is provided a fusion polypeptide wherein said tag moiety is fused, optionally via a linker, to an amino acid residue position corresponding to fusion site 1, 2 or 3 of SEQ ID NO:283, wherein fusion site 1 corresponds to the amino acid residues 1-12 corresponding to sequence KVEQAVETEPEP (SEQ ID NO:385), fusion site 2 corresponds to the amino acid residues 42-55 corresponding to sequence TLSEQVQEELLSSQ (SEQ ID NO:386), and fusion site 3 corresponds to the amino acid residues 162-201 corresponding to sequence YQAGAREGAERGLSAIRERLGPLVEQGRVRAATVGSLAGQ (SEQ ID NO:388) of ApoE isoform 2 (SEQ ID NO:283), and wherein all amino acid residues N-terminal to said amino acid residue position are not present. For the sake of clarity, the wording "fused, optionally via a linker, to an amino acid residue position corresponding to fusion site 1, 2 or 3" is to be understood as fused to any amino acid residues within said fusion site. The skilled person will appreciate that amino acid residue positions corresponding to fusion site 1, 2 or 3 of SEQ ID NO:283 can be found in any one of the ApoE2 mutated variants as defined in SEQ ID NO: 389, 18, 391-401. In this context, fusion site 1, 2 or 3 of SEQ ID NO:283 correspond to fusion site 1, 2 or 3 of SEQ ID NO:18.

In one particular embodiment, said apolipoprotein or fragment thereof is selected from the group consisting of SEQ ID NO: 18, SEQ ID NO:19, SEQ ID NO:20, SEQ ID NO:21, SEQ ID NO:22, SEQ ID NO:283and SEQ ID NO:286.

Thus, in one particular embodiment, said tag moiety as defined herein is fused, optionally via a linker, to apolipoprotein or fragment thereof having an amino acid sequence selected from the group consisting of SEQ ID NO:18, SEQ ID NO:19, SEQ ID NO:20, SEQ ID NO:21, SEQ ID NO:22 and SEQ ID NO:286, such as to a apolipoprotein fragment having an amino acid sequence selected from the group consisting of SEQ ID NO:19, SEQ ID NO:20, SEQ ID NO:21, SEQ ID NO:22 and SEQ ID NO:286.ln one embodiment there is provided a fusion polypeptide, wherein said tag moiety is fused, optionally via a linker, to apolipoprotein or fragment thereof having an amino acid sequence selected from the group consisting of SEQ ID NO:18 and SEQ ID NO:19, and said tag moiety is SEQ ID NO: 2 or 3. Said linker may be the flexible linker SEQ ID NO:9. Said linker may be the rigid linker SEQ ID NO:12. Said linker may be the cleavable linker SEQ ID NO:328.

Thus in one embodiment, said fusion polypeptide comprises SEQ ID NO:2 and SEQ ID NO:18. In one embodiment, said fusion polypeptide comprises SEQ ID NO:2, SEQ NO:9 and SEQ ID NO:18. In one embodiment, said fusion polypeptide comprises SEQ ID NO:2, SEQ NO:12 and SEQ ID NO:18. In one embodiment, said fusion polypeptide comprises SEQ ID NO:2, SEQ NO:328 and SEQ ID NO:18.

Thus in one embodiment, said fusion polypeptide comprises SEQ ID NO:3 and SEQ ID NO:18. In one embodiment, said fusion polypeptide comprises SEQ ID NO:3, SEQ NO:9 and SEQ ID NO:18. In one embodiment, said fusion polypeptide comprises SEQ ID NO:3, SEQ NO:12 and SEQ ID NO:18. In one embodiment, said fusion polypeptide comprises SEQ ID NO:3, SEQ NO:328 and SEQ ID NO:18.

Thus in one embodiment, said fusion polypeptide comprises SEQ ID NO:2 and SEQ ID NO:19. In one embodiment, said fusion polypeptide comprises SEQ ID NO:2, SEQ NO:9 and SEQ ID NO:19. In one embodiment, said fusion polypeptide comprises SEQ ID NO:2, SEQ NO:12 and SEQ ID NO:19. In one embodiment, said fusion polypeptide comprises SEQ ID NO:2, SEQ NO:328 and SEQ ID NO:19.

Thus in one embodiment, said fusion polypeptide comprises SEQ ID NO:3 and SEQ ID NO:19. In one embodiment, said fusion polypeptide comprises SEQ ID NO:3, SEQ NO:9 and SEQ ID NO:19. In one embodiment, said fusion polypeptide comprises SEQ ID NO:3, SEQ NO:12 and SEQ ID NO:19. In one embodiment, said fusion polypeptide comprises SEQ ID NO:3, SEQ NO:328 and SEQ ID NO:19.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:18.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:18. In one embodiment, said fusion polypeptide does not comprise a linker. In one embodiment said fusion polypeptide comprises or consists of an amino acid sequence as defined in SEQ ID NO:33. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO:18. Said linker may be selected from the group consisting of SEQ ID NO:9-17 and 328-330. In one embodiment said fusion polypeptide is selected from the group consisting of SEQ ID NO 34-42 and SEQ ID NO:331-333. Said linker may be selected from the group consisting of SEQ ID NO:9, 12 and 328. In one embodiment said fusion polypeptide is selected from the group consisting of SEQ ID NO: 34, 37 and 331.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO:18. In one embodiment, said fusion polypeptide does not comprise a linker. In one embodiment said fusion polypeptide comprises or consists of an amino acid sequence as defined in SEQ ID NO:43. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO:18. Said linker may be selected from the group consisting of SEQ ID NO:9-17 and 328-330. In one embodiment said fusion polypeptide is selected from the group consisting of SEQ ID NO 44-52 and SEQ ID NO:349-351. Said linker may be selected from the group consisting of SEQ ID NO:9, 12 and 328. In one embodiment said fusion polypeptide is selected from the group consisting of SEQ ID NO: 44, 47 and 349.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO:18. In one embodiment, said fusion polypeptide does not comprise a linker. In one embodiment said fusion polypeptide comprises or consists of an amino acid sequence as defined in SEQ ID NO:53. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO:18. Said linker may be selected from the group consisting of SEQ ID NO:9-17 and 328-330. In one embodiment said fusion polypeptide is selected from the group consisting of SEQ ID NO 54-62 and SEQ ID NO:367-369.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:19.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:19. In one embodiment, said fusion polypeptide does not comprise a linker. In one embodiment said fusion polypeptide comprises or consists of an amino acid sequence as defined in SEQ ID NO:74. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO:19. Said linker may be selected from the group consisting of SEQ ID NO:9-17 and 328-330. In one embodiment said fusion polypeptide is selected from the group consisting of SEQ ID NO: 75-83 and SEQ ID NO:334-336. Said linker may be selected from the group consisting of SEQ ID NO:9, 12 and 328. In one embodiment, said fusion polypeptide is selected from the group consisting of SEQ ID NO: 75, 78 and 335.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO:19. In one embodiment, said fusion polypeptide does not comprise a linker. In one embodiment said fusion polypeptide comprises or consists of an amino acid sequence as defined in SEQ ID NO:84. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO:19. Said linker may be selected from the group consisting of SEQ ID NO:9-17 and 328-330. In one embodiment said fusion polypeptide is selected from the group consisting of SEQ ID NO: 85-93 and SEQ ID NO:352-354. Said linker may be selected from the group consisting of SEQ ID NO:9, 12 and 328. In one embodiment, said fusion polypeptide is selected from the group consisting of SEQ ID NO: 55, 88 and 352.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO:19. In one embodiment, said fusion polypeptide does not comprise a linker. In one embodiment said fusion polypeptide comprises or consists of an amino acid sequence as defined in SEQ ID NO:94. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO:19. Said linker may be selected from the group consisting of SEQ ID NO:9-17 and 328-330. In one embodiment said fusion polypeptide is selected from the group consisting of SEQ ID NO: 95-103 and SEQ ID NO:370-372.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:20.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:20. In one embodiment, said fusion polypeptide does not comprise a linker. In one embodiment said fusion polypeptide comprises or consists of an amino acid sequence as defined in SEQ ID NO:115. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO:20. Said linker may be selected from the group consisting of SEQ ID NO:9-17 and 328-330. In one embodiment said fusion polypeptide is selected from the group consisting of SEQ ID NO: 116-124 and SEQ ID NO:337-339.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO:20. In one embodiment, said fusion polypeptide does not comprise a linker. In one embodiment said fusion polypeptide comprises or consists of an amino acid sequence as defined in SEQ ID NO:125. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO:20. Said linker may be selected from the group consisting of SEQ ID NO:9-17 and 328-330. In one embodiment said fusion polypeptide is selected from the group consisting of SEQ ID NO: 126-134 and SEQ ID NO:355-357.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO:20. In one embodiment, said fusion polypeptide does not comprise a linker. In one embodiment said fusion polypeptide comprises or consists of an amino acid sequence as defined in SEQ ID NO:135. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO:20. Said linker may be selected from the group consisting of SEQ ID NO:9-17 and 328-330. In one embodiment said fusion polypeptide is selected from the group consisting of SEQ ID NO: 136-144 and SEQ ID NO:373-375.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:21.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:21. In one embodiment, said fusion polypeptide does not comprise a linker. In one embodiment said fusion polypeptide comprises or consists of an amino acid sequence as defined in SEQ ID NO:156. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO:21. Said linker may be selected from the group consisting of SEQ ID NO:9-17 and 328-330. In one embodiment said fusion polypeptide is selected from the group consisting of SEQ ID NO 157-165 and SEQ ID NO:337-339.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO:21. In one embodiment, said fusion polypeptide does not comprise a linker. In one embodiment said fusion polypeptide comprises or consists of an amino acid sequence as defined in SEQ ID NO:166. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO:21. Said linker may be selected from the group consisting of SEQ ID NO:9-17 and 328-330. In one embodiment said fusion polypeptide is selected from the group consisting of SEQ ID NO 167-175 and SEQ ID NO:358-360.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO:21. In one embodiment, said fusion polypeptide does not comprise a linker. In one embodiment said fusion polypeptide comprises or consists of an amino acid sequence as defined in SEQ ID NO:176. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO:21. Said linker may be selected from the group consisting of SEQ ID NO:9-17 and 328-330. In one embodiment said fusion polypeptide is selected from the group consisting of SEQ ID NO 177-186 and SEQ ID NO:373-375.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:22.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:22. In one embodiment, said fusion polypeptide does not comprise a linker. In one embodiment said fusion polypeptide comprises or consists of an amino acid sequence as defined in SEQ ID NO:197. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO:22. Said linker may be selected from the group consisting of SEQ ID NO:9-17 and 328-330. In one embodiment said fusion polypeptide is selected from the group consisting of SEQ ID NO: 198-206 and SEQ ID NO:343-345.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO:22. In one embodiment, said fusion polypeptide does not comprise a linker. In one embodiment said fusion polypeptide comprises or consists of an amino acid sequence as defined in SEQ ID NO:207. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO:22. Said linker may be selected from the group consisting of SEQ ID NO:9-17 and 328-330. In one embodiment said fusion polypeptide is selected from the group consisting of SEQ ID NO 208-216 and SEQ ID NO:361-363.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO:22. In one embodiment, said fusion polypeptide does not comprise a linker. In one embodiment said fusion polypeptide comprises or consists of an amino acid sequence as defined in SEQ ID NO:217. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO:22. Said linker may be selected from the group consisting of SEQ ID NO:9-17 and 328-330. In one embodiment said fusion polypeptide is selected from the group consisting of SEQ ID NO 177-186 and SEQ ID NO:379-381.

In one embodiment of the fusion polypeptide as defined herein, said tag moiety comprises or consists of an amino acid sequence selected from the group consisting of SEQ ID NO:2-7, such as the group consisting of SEQ ID NO:2-4, such as is SEQ ID NO:3, and said apolipoprotein or fragment thereof comprises or consists of SEQ ID NO:286.

In one embodiment of the fusion polypeptide as defined herein, said tag moiety comprises or consists of SEQ ID NO:2. In one embodiment, said fusion polypeptide said apolipoprotein or fragment thereof comprises or consists of SEQ ID NO:286. In one embodiment, said fusion polypeptide does not comprise a linker. In one embodiment said fusion polypeptide comprises or consists of an amino acid sequence as defined in SEQ ID NO:297. In one embodiment, said fusion polypeptide comprises a linker. In one embodiment, said fusion polypeptide as defined herein comprises a tag moiety as defined in SEQ ID NO:2 and an apolipoprotein or fragment thereof as defined in SEQ ID NO: 286. Said linker may be selected from the group consisting of SEQ ID NO:9-17 and 328-330. In one embodiment said fusion polypeptide is selected from the group consisting of SEQ ID NO 298-306 and SEQ ID NO:346-348.

In one embodiment of the fusion polypeptide as defined herein, said tag moiety comprises or consists of SEQ ID NO:3. In one embodiment, said fusion polypeptide said apolipoprotein or fragment thereof comprises or consists of SEQ ID NO: 286. In one embodiment, said fusion polypeptide does not comprise a linker. In one embodiment said fusion polypeptide comprises or consists of an amino acid sequence as defined in SEQ ID NO:307. In one embodiment, said fusion polypeptide comprises a linker. In one embodiment, said fusion polypeptide as defined herein comprises a tag moiety as defined in SEQ ID NO:3 and an apolipoprotein or fragment thereof as defined in SEQ ID NO: 286. Said linker may be selected from the group consisting of SEQ ID NO:9-17 and 328-330. In one embodiment said fusion polypeptide is selected from the group consisting of SEQ ID NO 308-316 and SEQ ID NO:364-366.

In one embodiment of the fusion polypeptide as defined herein, said tag moiety comprises or consists of SEQ ID NO:4. In one embodiment, said fusion polypeptide said apolipoprotein or fragment thereof comprises or consists of SEQ ID NO: 286. In one embodiment, said fusion polypeptide does not comprise a linker. In one embodiment said fusion polypeptide comprises or consists of an amino acid sequence as defined in SEQ ID NO:317. In one embodiment, said fusion polypeptide comprises a linker. In one embodiment, said fusion polypeptide as defined herein comprises a tag moiety as defined in SEQ ID NO:4 and an apolipoprotein or fragment thereof as defined in SEQ ID NO: 286. Said linker may be selected from the group consisting of SEQ ID NO:9-17 and 328-330. In one embodiment said fusion polypeptide is selected from the group consisting of SEQ ID NO 318-326 and SEQ ID NO:382-384.

In one embodiment, said fusion polypeptide is a fusion polypeptide comprising an amino acid sequence selected from the group consisting of SEQ ID NO:33-62, 74-103, 115-144, 156-185, 197-226, 297-326 and 331-384, such as the group consisting of SEQ ID NO:33-62, 74-103 and 331-336. In one embodiment, said fusion polypeptide as defined herein, wherein said fusion polypeptide is a fusion polypeptide comprising an amino acid sequence selected from the group consisting of SEQ ID NO: 33, 43, 53, 74, 84, 94, 115, 125,, 156, 166, 176, 197, 207, 217, 297, 307 and 317, such as the group consisting of SEQ ID NO:33, 43, 74, 84, 115, 125, 156, 197, 207, 297 and 307, such as the group consisting of SEQ ID NO:33, 43, 74 and 84. In one embodiment, said fusion polypeptide is a fusion polypeptide comprising an amino acid sequence selected from the group consisting of SEQ ID NO: 33 and 43. In one embodiment, said fusion polypeptide is a fusion polypeptide comprising an amino acid sequence selected from the group consisting of SEQ ID NO: 74 and 84. [preferred embodiments]. In one particular embodiment, said fusion polypeptide is a fusion polypeptide comprising an amino acid sequence selected from the group consisting of SEQ ID NO: 43-54, 84-93, 125-134, 166-175, 207-216, 307-316 and 349-366 , such as the group consisting of SEQ ID NO:43, 84, 125, 166, 207 and 307.

In one embodiment, said fusion polypeptide is a fusion polypeptide comprising an amino acid sequence selected from the group consisting of SEQ ID NO: 33, 34, 37, 43, 44, 47, 74, 75, 78, 84, 85, 88, 331, 335, 344 and 352. In one embodiment, said fusion polypeptide is a fusion polypeptide comprising an amino acid sequence selected from the group consisting of SEQ ID NO: 34, 37, 44, 47, 75, 78, 85, 88, 331, 335, 344 and 352. In one embodiment, said fusion polypeptide is selected from the group consisting of SEQ ID NO:33, 34, 37 and 331; or the group consisting of SEQ ID NO:74, 75, 78 and 335; or the group consisting of SEQ ID NO:43, 44, 47 and 349; or the group consisting of SEQ ID NO:84, 85, 88 and 352. In one embodiment, said fusion polypeptide is selected from the group consisting of SEQ ID NO:34, 37 and 331; or the group consisting of SEQ ID NO:75, 78 and 335; or the group consisting of SEQ ID NO:44, 47 and 349; or the group consisting of SEQ ID NO:85, 88 and 352. In one embodiment, said fusion polypeptide is a fusion polypeptide comprising an amino acid sequence selected from the group consisting of SEQ ID NO:34, 37 and 331; or the group consisting of SEQ ID NO:75, 78 and 335; or the group consisting of SEQ ID NO:44, 47 and 349; or the group consisting of SEQ ID NO:85, 88 and 352.

Liponanoparticles (LNPs), also referred to as lipid nanoparticles, have emerged across the pharmaceutical industry as promising vehicles to deliver a variety of therapeutics. For the sake of clarity, the terms liponanoparticles and lipid nanoparticles are used interchangeably herein. An LNP is typically spherical with an average diameter of from about 10 to about 1000 nm, such as from about 50 to about 250 nm, such as from about 50 to about 200 nm, such as from about 80 to about 140 nm.

Solid LNPs possess a solid lipid core matrix that can solubilize lipophilic molecules. A solid LNP is generally spherical in shape and consists of a solid lipid core stabilized by a surfactant. The core lipids can be fatty acids, acylglycerols, waxes, and mixtures of these surfactants. Biological membrane lipids such as phospholipids, sphingomyelins, bile salts (sodium taurocholate), and sterols (cholesterol) may be utilized as stabilizers. Biological lipids having minimum carrier cytotoxicity and the solid state of the lipid permit better controlled drug release due to increased mass transfer resistance.

LNPs have been described as a platform nanoparticle technology which is suitable for delivery of payload, such as drug payload, to a subject in need thereof. Of particular interest is the targeted delivery of the payload to organs, tissues and/or cells of interest in the subject in need thereof. The present inventors have invented a system for the targeted delivery of payload, by utilizing a fusion polypeptide comprising a tag moiety, which is suitable due to its affinity for a specific binding molecule that may be part of a targeting component, and an apolipoprotein or fragment thereof coupled to the LNP. This system allows for the targeted delivery of a payload of interest in a targeted manner.

In particular, said payload may be a nucleic acid therapeutic (NAT), but LNPs are also useful for the delivery of other payloads as discussed below. The nanoparticles may be (phospho)lipid-based nanoparticles comprising apolipoproteins or fragments thereof and protect the NAT payload in the circulation by preventing it from degradation and rapid clearance. At the same time, the nanoparticles may reduce NAT's immunostimulatory related adverse effects by limiting unwanted interactions with components in the blood. Throughout this disclosure, a liponanoparticle acting as a payload delivery vessel and therein comprising a payload is also referred to as a "loaded liponanoparticle". Furthermore, if a liponanoparticle comprises at least one fusion polypeptide as defined herein, such as a fusion polypeptide coupled to said LNP's surface layer by means of the lipid binding domain of the apolipoprotein or fragment thereof comprised in said fusion polypeptide, then the liponanoparticle is also referred to as a "decorated liponanoparticle". If a liponanoparticle comprises a core which comprises a payload, and said liponanoparticle further comprises at least one fusion polypeptide as described above, then the liponanoparticle is also referred to as a "loaded decorated liponanoparticle".

Thus, in the second aspect of the present disclosure, there is provided a loaded decorated liponanoparticle comprising a core surrounded by a surface layer, wherein said core comprises at least one payload and said surface layer comprises: at least one phospholipid; and
at least one fusion polypeptide as defined herein.

In one embodiment, said loaded decorated liponanoparticle comprises at least one lipid, at least one triglyceride and/or at least one sterol, such as cholesterol.

Said lipid may be at least one cationic lipid. In one embodiment, said loaded decorated liponanoparticle comprises for further comprises (a) at least one cationic lipid; (b) at least one non-cationic lipid; and/or (c) at least one lipid conjugate , wherein said at least one cationic lipid, said at least one non-cationic lipid, and/or said at least one lipid conjugate is optionally chemically modified, such as chemically modified at its terminus. Chemical modification can be pegylation.

In one particular embodiment, said loaded decorated liponanoparticle comprises at least one ionizable cationic lipid.

In one embodiment, said loaded decorated liponanoparticle comprises 1,2-distearoyl-sn-glycero-3-phosphocholine (DSPC), 1,2-dioleoyl-sn-glycero-3-phosphoethanolamine (DOPE) and/or 1,2-dioleoyl-sn-glycero-3-phosphocholine (DOPC)

In one particular embodiment, said loaded decorated liponanoparticle comprises cholesterol.

In one particular embodiment, said loaded decorated liponanoparticle comprises polyethylene glycol (PEG) , such as in the form of a PEG-lipid conjugate.

In one particular embodiment, said loaded decorated liponanoparticle comprises at last one ionizable cationic lipid, 1,2-distearoyl-sn-glycero-3-phosphocholine (DSPC), cholesterol and polyethylene glycol, such as in the form of a PEG-lipid conjugate.

In one embodiment, said apolipoprotein or fragment thereof is bound to one component of the loaded decorated liponanoparticle. Said apolipoprotein or fragment thereof may be bound by hydrophobic bonds, hydrophilic bonds or non-covalent bonds. Said apolipoprotein or fragment thereof is bound by covalent bonds.

In one embodiment, said apolipoprotein or fragment thereof is covalently bound to said at least one cationic lipid, is covalently bound to said at least one non-cationic lipid, or is covalently bound to said at least one lipid conjugate; such as is covalently bound to the terminus of said at least one cationic lipid, such as is covalently bound to the terminus of said at least one non-cationic lipid, or such as is covalently bound to the terminus of said at least one lipid conjugate, wherein optionally said terminus is chemically modified. Chemical modification can be pegylation.

As explained above, the loaded decorated liponanoparticle as defined herein, comprises a core comprising at least one payload. It will be appreciated that the loaded decorated liponanoparticle may be loaded with one or several different payloads. The payload may be a therapeutically and/or a prophylactically active payload, which upon delivery to a target cell, tissue or organ, exerts its therapeutic and/or prophylactic activity. The skilled person will appreciate that the payload may be of various kind that are to be delivered to the cytoplasm of a cell. For example peptide therapeutics of various sorts, chemical substances of various sorts and other drug entities. The payload may be one or several small molecular substances, chemical substances, chemotherapeutics, engagers, degraders, peptide therapeutics, mimetic drug, enzymes, lipid derivatives as well as nucleic acid therapeutics. Thus in one embodiment, said payload is selected from the group consisting of small molecular substances, chemical substances, chemotherapeutics, degraders, peptide therapeutics, mimetic drug, enzymes, lipid derivatives and nucleic acid therapeutics.

As used herein, the term "degraders" refers to drugs enabling the engagement of the ubiquitin-proteasome system to degrade a target proteins in a cell as part of a therapeutic mechanism.

In one embodiment, said payload is a nucleic acid therapeutic (NAT) payload. Many different types of RNA, DNA or synthetic oligonucleotides have been used as nucleic acid therapeutics. The present invention is not limited to a specific type of nucleic acid therapeutic. Thus, in an embodiment, the nucleic acid payload selected from the group consisting of RNA, DNA and nucleic acid analogs.

In one embodiment, said RNA is selected from the group consisting of microRNA (miRNA) , small interfering RNA (siRNA), piwi-interacting RNA (piRNA), small nuclear RNA (snoRNA), transfer RNA (tRNA), tRNA-derived small RNA (tsRNA), small regulatory RNA (srRNA), messenger RNA (mRNA), modified mRNA, ribosomal RNA (rRNA), long non-coding RNA (IncRNA) or guide RNA (gRNA) or combinations thereof and/or modifications thereof, such as wherein said RNA is messenger RNA (mRNA). In one embodiment, said payload is antisense oligonucleotide.

In one embodiment, said antisense oligonucleotide is single strand DNA or RNA.

The skilled person appreciates that the NAT payload, for example RNA or DNA payload, may be codon optimized and/or comprise modified nucleosides, such as naturally modified nucleotides and/or synthetic nucleoside analogues.

The skilled person is familiar with the concept of codon optimization and knows that it refers to experimental approaches designed to improve the codon composition of a recombinant nucleic acid based on various criteria without altering the amino acid sequence encoded by the nucleic acid. In the present context, the term "codon-optimized" refers to nucleic acid sequences comprising modified codons compared to the native naturally occurring sequences.

Nucleoside modified mRNAs encode the same protein as the protein encoded by the non-nucleoside modified mRNA variant. Certain modified nucleosides are considered to enhance the stability of the mRNA and/or increase the efficiency of its translation which in turn enhances the production of the desired protein in the cell comprising the mRNA. Certain nucleoside modifications are considered to alter the immunogenicity profile of the payload which comprise such nucleoside modified mRNA, and can be particularly useful to reduce the innate immune response often elicited by non-nucleoside modified mRNA variants. This may be achieved by decreasing the recognition of the mRNA as foreign by pattern recognition receptors which would normally result in the activation of innate immunity. Commonly employed nucleoside modifications include for example 5-methoxyuridine, which is a modified nucleoside triphosphate (NTP) for incorporation into mRNA using T7 RNA polymerase. Incorporation of 5-methoxyuridine can reduce the immunogenicity of the resulting mRNA. 5-methylcytidine (m5C) is also one example of such nucleoside modifications that involves the addition of a methyl group to cytidine. Additional non-limiting examples are N6-methyladenosine (m6A), which involves the addition of a methyl group to adenosine, 5-methyluridine (m5U), which involves the addition of a methyl group to uridine, 2-thiouridine (s2U), which contains a sulfur group and is known to increase mRNA stability, 2'-O-methylguanosine (m2,2G), which involves the addition of a methyl group to the 2'-oxygen of guanosine, 2'-O-methylcytidine (m2,2C), which involves the addition of a methyl group to the 2'-oxygen of cytidine, 2'-O-methyluridine (m2,2U), which involves the addition of a methyl group to uridine at the 2'-oxygen, and 5-methoxycytidine (m5oC), which contains a methoxy group. Pseudouridine (abbreviated by the Greek letter psi, ψ) is an isomer of the nucleoside uridine in which the uracil is attached via a carbon-carbon instead of a nitrogen-carbon glycosidic bond. Pseudouridine, which is the most abundant RNA modification in cellular RNA, can regulate RNA expression post-transcriptionally and plays a variety of roles in the cell including translation, localization and stabilization of RNA. Without being bound by theory, the present inventors envision that modified nucleosides which have one or more of the above discussed beneficial effects on mRNA stability, translation efficiency and/or immunogenicity profile are advantageous in the context of the payload of the present disclosure.

As used herein, the term "nucleoside modified" in relation to a nucleic acid sequence is meant to be understood that said nucleic acid sequence comprises at least one nucleoside modification as discussed above. In one non-limiting example, at least one uridine in an RNA sequence of said payload is replaced by pseudouridine. In another non-limiting example, all uridines in the RNA sequence of said payload are replaced by pseudouridines. In one embodiment, said one or more modified nucleoside(s) is/are selected from a group consisting of 5-methoxyuridine, 5-methylcytidine (m5C), N6-methyladenosine (m6A), 5-methyluridine (m5U), 2-thiouridine (s2U), 2'-O-methylguanosine (m2,2G), 2'-O-methylcytidine (m2,2C), 2'-O-methyluridine (m2,2U), 5-methoxycytidine (m5oC), 1-methylpseudouridine (m1ψ), 1-methyl-3-(3-amino-5-carboxypropyl)pseudouridine (m1acp3ψ), 2'-0-methylpseudouridine (ψm), 5-methyldihydrouridine (m5D) and (3-methylpseudouridine (m3ψ).

In one embodiment, said nucleic acid is an antisense oligonucleotide and the antisense oligonucleotide is single strand DNA or RNA consisting of nucleotide or nucleoside analog containing modifications of the phosphodiester backbone or the 2' ribose.

In another embodiment, said DNA is single stranded or double stranded DNA.

In one embodiment, said nucleotide or nucleoside analog is selected from the group consisting of locked nucleic acid (LNA), bridged nucleic acid (BNA), morpholino or peptide nucleic acid (PNA).

In the third aspect of the present disclosure there is provided a complex comprising a bispecific binding molecule as defined below and a loaded decorated liponanoparticle, said complex formed via binding of the bispecific binding molecule to the tag moiety on the fusion polypeptide, which is part of the loaded decorated liponanoparticle as described herein, via a second specific binding molecule.

Thus, in one embodiment according to the third aspect there is provided a complex comprising a bispecific binding molecule and a loaded decorated liponanoparticle, wherein said bispecific binding molecule comprises:
i) at least one first specific binding molecule with affinity for a target antigen;
ii) at least one second specific binding molecule with affinity for a tag moiety as defined herein,

wherein said first specific binding molecule and said second specific binding molecule are antigen-binding proteins each comprising an antigen-binding domain of an antibody and are covalently linked,
wherein said loaded decorated liponanoparticle is as defined herein and is non-covalently bound to said second specific binding molecule via said tag moiety. For the sake of clarity, it is to be understood that the loaded decorated liponanoparticle referred to above, is a loaded decorated liponanoparticle according to the second aspect as disclosed herein, which comprises the fusion polypeptide according to the first aspect.

Since the tag moiety of said fusion polypeptide is non-covalently bound to the second specific binding molecule of said bispecific binding molecule, the complex provides flexibility in what payload may be delivered to the tissue, organ or cell of interest. By having affinity for the tag moiety and thus being able to bind to the tag moiety, the second specific binding molecule may be bound indirectly to the loaded decorated liponanoparticle and thereby provide a flexible approach by which the loaded decorated liponanoparticle and the payload comprised therein can easily be varied, since there is no chemical linkage between them and the bispecific binding molecule.

The skilled person appreciates that the design considerations for creating a bispecific binding molecule, also referred to as a bispecific conjugate, of this aspect of the disclosure are analogous to what is described for similar conjugates in WO2020/104690 and WO2021/239968. The bispecific binding molecules referred to herein may be specific binding proteins, and more particularly they may be antigen-binding proteins. The term "antigen-binding protein" is used herein to denote a binding protein comprising an antigen-binding domain obtained or derived from an antibody, or based on an antigen binding domain of an antibody. Thus, the antigen-binding protein may be an antibody-based, or antibody-like, molecule comprising the binding site of, or a binding site derived from, an antibody. An antigen-binding protein may thus be a native antibody or a fragment thereof, or an artificial or synthetic antibody, or an antibody construct, or derivative (e.g. a single chain antibody, as discussed further below).

Herein, the first specific binding molecule is an antibody or antigen binding fragment thereof with a binding affinity for an immunologically relevant target, and the second moiety of the bispecific binding molecule is a binding molecule, which is advantageously capable of binding to a tag moiety.

The skilled person appreciates that since the first specific binding molecule comprised in the bispecific binding molecule is an antibody or antigen binding fragment thereof with a binding affinity for an immunologically relevant target, it can be varied, allowing the bispecific binding molecule to be tailored to target a specific and relevant target depending on the application of the complex. Examples of relevant targets for the first specific binding molecule could be a target antigen, lipid structure, or carbohydrate. An alternative target could be unnatural molecules such as chemical structures.

In one embodiment, said complex as defined herein comprises said bispecific binding molecule as defined herein, wherein said first specific binding molecule has affinity for CD40. In particular the first specific binding molecule may have affinity for CD40 when localized on the surface of a cell, more particularly an antigen-presenting cell. The CD40 may be human CD40. CD40 is a member of the TNF receptor superfamily and is expressed on antigen presenting cells (APC) such as B-cells, dendritic cells, macrophages and monocytes, as well as epithelial and endothelial cells and certain tumor cells. When activated by its ligand (CD40 ligand; also known as CD154), which is mainly expressed on mature T-cells, CD40 activates APC and induces both innate and adaptive immune responses. Agonistic CD40 agents, such as anti-CD40 antibodies, have been described to be used to induce the immune system to prevent proliferation of and/or kill cancer cells and thus provide an effective therapeutic treatment for cancer. In particular, APC activated by an agonistic CD40 agent may stimulate T-cells, including effector T-cells, particularly CD8+ cytotoxic T-cells, leading to T-cell mediated destruction of cancer cells (e.g. tumors). Thus, in one embodiment, said complex as defined herein comprises said bispecific binding molecule as defined herein, wherein said first specific binding molecule is an agonist of CD40.

As is well known in the art, CD40 is a cell surface protein and upon engagement by a ligand or agonist, CD40 may be internalized. Anti-CD40 antibodies may have different internalization rates. Without being bound by theory, it is possible that antibody internalization is linked to agonistic capacity.

However, it is not considered that internalization and agonistic effect are necessarily coupled. It is also possible that the first specific binding molecule leads to the internalization of CD40 upon binding to CD40 without having an agonistic effect. Thus, in one embodiment of the complex as disclosed herein, said first specific binding molecule induces CD40 internalization upon binding to CD40. Internalization of said complex, wherein said at least one first specific binding molecule has affinity for CD40, results in internalization by the target cell of the loaded decorated liponanoparticle as defined herein, which is bound via the tag moiety to the second specific binding molecule. It is considered that the internalization may promote antigen cross-presentation.

The term "antibody" as used herein refers to an immunoglobulin molecule.

An antibody thus comprises at least two heavy (H) chains and two light (L) chains inter-connected by disulphide bonds. Each heavy chain is comprised of a heavy chain variable region (VH) and a heavy chain constant region. Each light chain is comprised of a light chain variable region (VL) and a light chain constant region. The VH and VL contain the binding domain that interacts with an antigen. The VH and VL can be sub-divided into regions of hypervariability, termed complementarity determining regions (CDR), interspersed with regions that are more conserved, termed framework regions (FR). Each VH and VL typically has 3 CDRs (which may be used to define the antigen-binding domain/site), and 4 frame work regions. The constant regions of the antibody may mediate the binding of the antibody to various cells or factors. The Fc region of the antibody is composed of parts of the constant regions of the two heavy chains (which contribute two or three constant domains to the Fc, depending on the class of the antibody), and this region in particular may bind to the Fc receptor which is present on certain cells, including APC, and may play a role in the ability of the antibody to stimulate the cell. An antibody may be a polyclonal or a monoclonal antibody. The antibody may be of any isotype (or class), which is determined by the type of constant domain in the heavy chains. Thus it may be an IgA, IgD, IgE, IgG, or IgM antibody. Several of these classes are further divided into subclasses, such as IgG1, IgG2, IgG3, IgG4, and the like, and the antibody may be of any subclass. The heavy-chain constant domains that correspond to the difference classes of immunoglobulins are termed α, δ, ε, γ and µ, respectively. The subunit structures and three-dimensional configurations of different classes of immunoglobulins are well known.

In some embodiments, the first specific binding molecule is an antibody or an antigen binding fragment thereof. For the avoidance of any doubt, when referring to an antibody or a fragment thereof, the term "a fragment thereof" is to be understood to mean an antigen binding fragment thereof. It may be an antibody comprising a full complement of H and L chain constant regions. In other embodiments the first specific binding molecule, may be an engineered or synthetic antibody construct. The first specific binding molecule may be an antigen-binding fragment of an antibody, which fragment which retains the ability of the antibody to bind specifically to an antigen. Such fragments are well known in the art and non-limiting examples thereof include Fab', Fab, F(ab')2, Fv, Fd, or dAb fragments and the skilled person knows how to prepare such fragments. It is also possible that said first specific binding molecule may be a synthetic or artificial construct, i.e. an antibody-like molecule which comprises an antigen-binding domain, but which is genetically engineered or artificially constructed. This includes chimeric or CDR-grafted antibodies, as well as single chain antibodies and other constructs. Non limiting example of such synthetic or artificial construct are scFv, dsFv, ds-scFv, dimers, minibodies, diabodies, single domain antibodies (DABs), TandAbs dimers, heavy chain antibodies such as VHH and camel antibodies . A scFv typically comprises, N-terminal to C-terminal, a VH region linked to a VL region by a linker sequence. Examples of suitable linkers in the context of a scFV are discussed below. In one specific embodiment of the complex as defined herein, said first specific binding molecule is an anti-CD40 antibody or antigen binding fragment thereof, such as an scFv. The first specific binding molecule may be human or humanized proteins antibody or antigen binding fragment thereof. The skilled person is familiar with humanization of antibodies and of fragments thereof.

In one embodiment, said anti-CD40 antibody is selected from the group consisting of CP-870,893, APX005M, ADC-1013, ChiLob 7/4, SEA-CD40 and ABS-1150/1151 and an antibody comprising an antigen binding fragment derived from any one or more of said antibodies. Said antibodies and the sequences thereof are disclosed in WO2020/104690 on pages 26-28 and incorporated herein by reference. It is envisioned that any one of said antibodies or fragments thereof may be used as the basis for preparing antibody derivatives to use as the first specific binding molecule with affinity for CD40.

In another embodiment, said anti-CD40 antibody or antigen binding fragment thereof comprises six complementarity determining domains (CDRs), wherein:
VLCDR1 has the sequence set forth in SEQ ID NO:259;
VLCDR2 has the sequence "AAS";
VLCDR3 has the sequence set forth in SEQ ID NO:261;
VHCDR1 has the sequence set forth in SEQ ID NO:262;
VHCDR2 has the sequence set forth in SEQ ID NO:263; and
VHCDR3 has the sequence set forth in SEQ ID NO:264.

In one embodiment there is provided a complex as disclosed herein, wherein the light chain variable domain of said anti-CD40 antibody comprises an amino acid sequence selected from SEQ ID NO:265 and amino acid sequences having at least 90%, such as at least 95%, sequence identity thereto; and the heavy chain variable domain of said anti-CD40 antibody comprises an amino acid sequence selected from SEQ ID NO:266 and amino acid sequences having at least 90 %, such as at least 95%, sequence identity thereto.

In an embodiment in which said anti-CD40 antibody or antigen binding fragment thereof is a full-length antibody, it may for example comprise a light chain (LC) comprising an amino acid sequence selected from SEQ ID NO:442 and amino acid sequences having at least 90%, such as at least 95%, sequence identity thereto; and a heavy chain (HC) comprising an amino acid sequence selected from SEQ ID NO:443 and amino acid sequences having at least 90%, such as at least 95%, sequence identity thereto. In one embodiment, one or more of said VH, VL, LC and HC sequences has at least 91%, such as at least 92%, such as at least 93%, such as at least 94%, such as at least 95%, such as at least 96%, such as at least 97%, such as at least 98%, such as at least 99% identity to the VH, VL, LC and HC sequences recited above. In another embodiment, the complex of the disclosure may comprise a bispecific binding molecule, wherein said bispecific binding molecule consists of two copies of a polypeptide chain comprising SEQ ID NO:446, which constitutes the heavy chain of the anti-CD40 antibody linked to an scFv, and two copies of a polypeptide chain comprising SEQ ID NO:442, which constitutes the light chain of the anti-CD40 antibody.

As described herein, binding molecules having amino acid sequences with 90 % or more identity to the listed sequences are also contemplated to fall within the ambit of the disclosure. Such variant sequences may be modified relative to the listed sequences by substitution, insertion and/or deletion of one or more amino acids. An amino acid substitution relative to a listed sequence may be a conservative amino acid substitution. The term "conservative amino acid substitution", as used herein, refers to an amino acid substitution in which one amino acid residue is replaced with another amino acid residue having a similar side chain. Amino acids with similar side chains tend to have similar properties, and thus a conservative substitution of an amino acid important for the structure or function of a polypeptide may be expected to affect polypeptide structure/function less than a non-conservative amino acid substitution at the same position. Families of amino acid residues having similar side chains have been defined in the art, including basic side chains (e.g. lysine, arginine, histidine), acidic side chains (e.g. aspartic acid, glutamic acid), uncharged polar side chains (e.g. asparagine, glutamine, serine, threonine, tyrosine), non-polar side chains (e.g. glycine, cysteine, alanine, valine, leucine, isoleucine, proline, phenylalanine, methionine, tryptophan) and aromatic side chains (e.g. tyrosine, phenylalanine, tryptophan, histidine). Thus, a conservative amino acid substitution may be considered to be a substitution in which a particular amino acid residue is substituted for a different amino acid in the same family. However, an amino acid substitution may equally be a non-conservative substitution, in which one amino acid is substituted for another with a side-chain belonging to a different family.

As detailed above, according to the present disclosure variants of the listed sequences have at least 90 % sequence identity to the respective listed sequence. Sequence identity may be assessed by any convenient method. However, for determining the degree of sequence identity between sequences, computer programs that make pairwise or multiple alignments of sequences are useful, for instance EMBOSS Needle or EMBOSS stretcher (both Rice et al. (2000), Trends Genet., 16(6):276-277) may be used for pairwise sequence alignments, while Clustal Omega (Sievers et al. (2011), Mol. Syst. Biol. 7:539) or MUSCLE (Edgar (2004), Nucleic Acids Res. 32(5):1792-1797) may be used for multiple sequence alignments, though any other appropriate program may be used. Whether the alignment is pairwise or multiple, it must be performed globally (i.e., across the entirety of the reference sequence) rather than locally. Sequence alignments and % identity calculations may be determined using for instance standard Clustal Omega parameters: matrix Gonnet, gap opening penalty 6, gap extension penalty 1. Alternatively, the standard EMBOSS Needle parameters may be used: matrix BLOSUM62, gap opening penalty 10, gap extension penalty 0.5. Any other suitable parameters may alternatively be used.

In another complex according to the third aspect as disclosed herein, the first specific binding molecule has affinity for a different target, such as human epidermal growth factor receptor 2 (HER2). HER2, also known as receptor tyrosine-protein kinase erbB-2 and CD340, is a protein that normally resides in the membranes of cells and is encoded by the ERBB2 gene and the over-expression thereof is associated with aggressive breast cancer. Over-expression of the HER2 occurs in approximately 15-30% of breast cancers and HER2-positive breast cancers are well established as being associated with increased disease recurrence and a poor prognosis and high risk of metastasis. It is of interest to specifically deliver a payload, such as an anti-cancer agent, to HER2-positive cells in patients in need thereof. Thus, in one embodiment there is provided a complex as defined herein, wherein first specific binding molecule has affinity for HER2. In one embodiment, said first specific binding molecule is an anti-HER2 antibody or an antigen-binding fragment thereof.

In one embodiment, said first specific binding molecule is an antibody selected from the group consisting of trastuzumab, pertuzumab, margetuximab and antigen-binding fragments thereof. In one particular embodiment, said first specific binding molecule is trastuzumab or an antigen-binding fragment thereof.

In one particular embodiment, said first specific binding molecule comprises a light chain (LC) variable domain comprising an amino acid sequence selected from SEQ ID NO:268 and amino acid sequences having at least 90%, such as at least 95%, sequence identity thereto; and a heavy chain (HC) variable domain comprising an amino acid sequence selected from SEQ ID NO:267 and amino acid sequences having at least 90%, such as at least 95%, sequence identity thereto. In one embodiment, said first specific binding molecule comprises LC variable domain and HC variable domain sequences which exhibit at least 91%, such as at least 92%, such as at least 93%, such as at least 94%, such as at least 95%, such as at least 96%, such as at least 97%, such as at least 98%, such as at least 99% identity to LC variable domain and HC variable domain sequences, respectively, as recited above. In one particular embodiment, said first specific binding molecule comprises a light chain (LC) comprising an amino acid sequence selected from SEQ ID NO:449 and amino acid sequences having at least 90%, such as at least 95%, sequence identity thereto; and a heavy chain (HC) comprising an amino acid sequence selected from SEQ ID NO:448 and amino acid sequences having at least 90%, such as at least 95%, sequence identity thereto. In one embodiment, said first specific binding molecule comprises LC and HC sequences which exhibit at least 91%, such as at least 92%, such as at least 93%, such as at least 94%, such as at least 95%, such as at least 96%, such as at least 97%, such as at least 98%, such as at least 99% identity to LC and HC sequences, respectively, as recited above. In another embodiment, the complex of the disclosure may comprise a bispecific binding molecule, wherein said bispecific binding molecule consists of two copies of a polypeptide chain comprising SEQ ID NO:450, which constitutes the heavy chain of the anti-HER2 antibody linked to an scFv, and two copies of a polypeptide chain comprising SEQ ID NO:449, which constitutes the light chain of the anti-HER2 antibody.

In another complex according to the third aspect as disclosed herein, the first specific binding molecule has affinity for a different target, such as CD20. CD20 is a transmembrane protein consisting of four hydrophobic transmembrane domains, one intracellular domain and two extracellular loops. CD20 is expressed on all stages of B cell development from pre-B cells in the bone-marrow through immature, naive, mature and memory cells in lymphoid tissues and blood. CD20 is known to be a marker of B cell malignancies and is amongst other found on B-cell lymphomas, hairy cell leukemia, B-cell chronic lymphocytic leukemia, and melanoma cancer stem cells. CD20 is also known to play a role in B-cell mediated autoimmune disease. Several antibodies that have affinity for CD20 are known in the art. Non-limiting examples thereof include rituximab, ocrelizumab, obinutuzumab, ofatumumab, ibritumomab, tiuxetan, tositumomab, and ublituximab.

Thus, in one embodiment there is provided a complex as defined herein, wherein first specific binding molecule has affinity for CD20. In one embodiment, said first specific binding molecule is an anti-CD20 antibody or an antigen-binding fragment thereof. In one embodiment, said first specific binding molecule is an antibody selected from the group consisting of rituximab, ocrelizumab, obinutuzumab, ofatumumab, ibritumomab tiuxetan, tositumomab, ublituximab and antigen-binding fragments thereof. In one particular embodiment, said first specific binding molecule is rituximab or an antigen-binding fragment thereof. In one particular embodiment, said first specific binding molecule comprises a light chain (LC) variable domain comprising an amino acid sequence selected from SEQ ID NO:270 and amino acid sequences having at least 90%, such as at least 95%, sequence identity thereto; and a heavy chain (HC) variable domain comprising an amino acid sequence selected from SEQ ID NO:269 and amino acid sequences having at least 90%, such as at least 95%, sequence identity thereto. In one embodiment, said first specific binding molecule comprises LC variable domain and HC variable domain sequences which exhibit at least 91%, such as at least 92%, such as at least 93%, such as at least 94%, such as at least 95%, such as at least 96%, such as at least 97%, such as at least 98%, such as at least 99% identity to LC variable domain and HC variable domain sequences, respectively, as recited above. In one particular embodiment, said first specific binding molecule comprises a light chain (LC) comprising an amino acid sequence selected from SEQ ID NO:452 and amino acid sequences having at least 90%, such as at least 95%, sequence identity thereto; and a heavy chain (HC) comprising an amino acid sequence selected from SEQ ID NO:451 and amino acid sequences having at least 90%, such as at least 95%, sequence identity thereto. In one embodiment, said first specific binding molecule comprises LC and HC sequences which exhibit at least 91%, such as at least 92%, such as at least 93%, such as at least 94%, such as at least 95%, such as at least 96%, such as at least 97%, such as at least 98%, such as at least 99% identity to LC and HC sequences, respectively, as recited above. In another embodiment, the complex of the disclosure may comprise a bispecific binding molecule, wherein said bispecific binding molecule consists of two copies of a polypeptide chain comprising SEQ ID NO:453, which constitutes the heavy chain of the anti-CD20 antibody linked to the scFv, and two copies of a polypeptide chain comprising SEQ ID NO:452, which constitutes the light chain of the anti-CD20 antibody.

In one embodiment, said complex as defined herein comprises said bispecific binding molecule as defined herein, wherein said second specific binding molecule is an antibody or an antigen binding fragment thereof. The term "binding molecule" is used herein to denote a second specific binding molecule comprising a binding domain of an antibody (that is to say, a binding domain obtained or derived from an antibody, or based on a binding domain of an antibody). Thus, the second specific binding molecule is an antibody-based, or antibody-like, molecule comprising the binding site of, or a binding site derived from, an antibody.

As noted above, a binding domain of an antibody is composed of a light chain variable domain and a heavy chain variable domain (thus a classical bivalent antibody has two binding domains). A second specific binding molecule may thus be a native antibody or a fragment thereof, or an artificial or synthetic antibody, or an antibody construct, or derivative (e.g. a single chain antibody). In summary, the second specific binding molecule of the disclosure comprises a binding domain of an antibody, said binding domain of an antibody comprising a light chain variable domain and a heavy chain variable domain.

In another embodiment, the complex of the disclosure may comprise a second specific binding molecule, wherein said second specific binding molecule comprises: an immunoglobulin heavy chain variable region (VH) comprising three complementarity determining domains (CDRs), wherein:
VHCDR1 has the sequence set forth in SEQ ID NO:252;
VHCDR2 has the sequence IGRIDPEXₐX_{b}DAEYVP (SEQ ID NO:253), wherein XₐX_{b} is selected from the group consisting of SG, GG, QG, DG, NA and NG and;
VHCDR3 has the sequence set forth in SEQ ID NO:254; and
an immunoglobulin light chain variable region (VL) comprising three complementarity determining domains (CDRs), wherein:
   VLCDR1 has the sequence set forth in SEQ ID NO:256;
   VLCDR2 has the sequence set forth in SEQ ID NO:257; and
   VLCDR3 has the sequence set forth in SEQ ID NO:258.

In another embodiment, the complex of the disclosure may comprise a second specific binding molecule , wherein said second specific binding molecule comprises an immunoglobulin heavy chain variable region (VH) consisting of an amino acid sequence selected from the group consisting of SEQ ID NO:246-251, and amino acid sequences having at least 90 % identity thereto; and an immunoglobulin light chain variable region (VL) consisting of an amino acid sequence set forth in SEQ ID NO:255, and amino acid sequences having at least 90 % identity thereto.

In another embodiment, the second specific binding molecule is a synthetic or artificial construct, i.e. an antibody-like molecule which comprises a binding domain, but which is genetically engineered or artificially constructed. Such constructs include chimeric or CDR-grafted antibodies, as well as single chain antibodies and other constructs, e.g. scFv, dsFv, ds-scFv, dimers, minibodies, diabodies, single domain antibodies (DABs), TandAbs dimers and heavy chain antibodies such as VHH, etc. In a preferred embodiment, said second specific binding molecule is a single chain variable fragment (scFv). An scFv is a fusion protein in which a single polypeptide comprises both the VH and VL domains of an antibody. scFv fragments generally include a peptide linker covalently joining the VH and VL regions, which contributes to the stability of the molecule. The linker may comprise from 1 to 20 amino acids, such as for example 1, 2, 3 or 4 amino acids, 5, 10 or 15 amino acids, or other intermediate numbers in the range 1 to 20 as convenient. The peptide linker may be formed from any generally convenient amino acid residues, such as glycine and/or serine. One example of a suitable linker is G₄S (SEQ ID NO:9). Multimers of such linkers may be used, such as for example a dimer, a trimer, a tetramer or a pentamer, i.e. (G₄S)₂ (SEQ ID NO:10), (G₄S)₃ (SEQ ID NO:11), (G₄S)₄ (SEQ ID NO:444) or (G₄S)₅ (SEQ ID NO:445). In a particular embodiment in which the binding molecule of the disclosure is an scFv, the linker is a (G₄S)₄ linker (SEQ ID NO:444). However, it is not essential that a linker be present, and the VL domain may instead be linked directly to the VH domain by a peptide bond. An scFv typically comprises, N-terminal to C-terminal, a VH region linked to a VL region by a linker sequence. In other words, in such an embodiment of a binding molecule of the disclosure, the scFv comprises a peptide linker joining the C terminus of the heavy chain variable region (VH) to the N terminus of the light chain variable region (VL). The preparation of scFv molecules is well known in the art, and a person of ordinary skill is able to readily prepare scFv binding molecules according to the disclosure which comprise the VH and VL sequences defined herein.

As described above, the binding molecule may in some embodiments be an scFv molecule, in which the VH and VL domains are joined together in a single polypeptide chain in a manner familiar to a person of skill in the art, e.g., via an amino acid linker. In one embodiment, the second specific binding molecule is an scFv comprising an amino acid sequence selected from the group consisting of SEQ ID NO:436-441. In a more specific embodiment, said amino acid sequence is selected from the group consisting of SEQ ID NO:436 and 441. In an even more particular embodiment, said amino acid sequence is SEQ ID NO:436.

The skilled person can readily produce binding proteins using appropriate methodology common in the art. In particular, the second specific binding molecule may be recombinantly expressed in mammalian cells, such as CHO cells. A second specific binding molecule synthesized in a protein expression system may be purified using standard techniques in the art, e.g. it may be synthesized with an affinity tag and purified by affinity chromatography. If the second specific binding molecule is an antibody, it can be purified using affinity chromatography using one or more antibody-binding proteins, such as Protein G, Protein A, Protein A/G or Protein L.

As noted above, the second specific binding molecule is an antibody-based, or antibody-like, molecule. Thus, a second specific binding molecule may be a native antibody or a fragment thereof, or an artificial or synthetic antibody, or an antibody construct or derivative (e.g. a single chain antibody).

The second specific binding molecule of the disclosure may be synthesized by any method known in the art. Preferably, the second specific binding molecule is synthesized using a protein expression system, such as a cellular expression system using prokaryotic (e.g. bacterial) cells or eukaryotic (e.g. yeast, fungus, insect or mammalian) cells. An alternative protein expression system is a cell-free, *in vitro* expression system, in which a nucleotide sequence encoding the second specific binding molecule is transcribed into mRNA, and the mRNA translated into a protein, *in vitro.* Cell-free expression system kits are widely available, and can be purchased from e.g. ThermoFisher. Alternatively, binding proteins may be chemically synthesized in a non-biological system. Liquid-phase synthesis or solid-phase synthesis may be used to generate polypeptides which may form or be comprised within the second specific binding molecule of the disclosure.

In one embodiment of the second specific binding molecule of the disclosure, it is capable of selective binding to a peptidic tag moiety comprised in a fusion polypeptide. By "selective binding" is meant that the second specific binding molecule binds to its target in a manner that can be distinguished from binding to non-target molecules, more particularly that the second specific binding molecule binds its target with greater binding affinity than with which it binds other molecules. That is, the second specific binding molecule does not bind to other, non-target, molecules, or does not do so to an appreciable or significant degree, or binds with lower affinity to such other molecules than with which it binds the tag moiety. A second specific binding molecule "that selectively binds" the tag moiety may alternatively be referred to as "directed against" or "recognizing" the tag moiety. In other words, the tag moiety is the antigen of the second specific binding molecule of the disclosure, and the second specific binding molecule is thus an "antigen binding protein" in the sense that it binds the tag moiety as its antigen. The skilled person will realize that the terms "antigen" and "antigen binding protein" in the previous sentence are applied in the context of a metaphor in an attempt to illustrate the binding relationship between the tag moiety and the second specific binding molecule of the disclosure. The skilled person will also realize that the previous sentence is not indicative of the tag moiety being an antigen itself which induces an unspecific or specific immune reaction towards it, but rather that the tag moiety is a bespoke binding partner to the second specific binding molecule of the disclosure.

In another embodiment, the complex of the disclosure may comprise a second specific binding molecule , wherein said second specific binding molecule is capable of biding to said tag moiety as defined herein such that the K_{D} value of the interaction is at most 1 × 10⁻⁹ M, for example at most 1 × 10⁻¹⁰ M, for example at most 1 × 10⁻¹¹ M.

In a fourth aspect of the disclosure, there is provided a polynucleotide encoding a fusion polypeptide as described herein; an expression vector comprising said polynucleotide; and a host cell comprising said expression vector. Said polynucleotide could also encode the bispecific binding molecule comprised in the complex as defined herein. Therein, in the fifth aspect of the disclosure, there is also provided polynucleotides encoding a bispecific binding molecule as described herein; an expression vector comprising one of two of said polynucleotides; and a host cell comprising said expression vector.

The polynucleotide according to any embodiment of this aspect may be DNA or RNA. A polynucleotide may encode one or more polypeptide chains. For example, a polynucleotide of the invention may encode a fusion polypeptide sequence or a sequence corresponding to a light chain, or a heavy chain of a bispecific binding molecule or both. Two polynucleotides may be provided, one of which encodes a light chain and the other of which encodes the corresponding heavy chain. Such a polynucleotide or pair of polynucleotides may be expressed together such that a conjugate is generated. A polynucleotide encoding a light and a heavy chain may encode the two chains separately (i.e. as separate genes under the control of two separate promoters and other expression control elements) or may encode them polycistronically. Alternatively, the two chains may be encoded as a single polypeptide separated by a self-splicing linker (such as a 2A linker, see Lewis et al., 2015, J Neurosci Methods 256: 22-29).

The polynucleotides of the invention can be synthesised according to methods well known in the art, as described by way of example in Sambrook et al. (1989, Molecular Cloning - a laboratory manual; Cold Spring Harbor Press).

The polynucleotides may be provided in the form of an expression cassette which includes control sequences operably linked to the encoding nucleotide sequence, thus allowing for expression of a fusion polypeptide or bispecific binding molecule in a protein expression system. These expression cassettes, in turn, are typically provided within vectors (e.g. plasmids or recombinant viral vectors). A suitable vector may be any vector which is capable of carrying a sufficient amount of genetic information, and allowing expression of a fusion polypeptide, bispecific binding molecule, conjugate or chain thereof.

The present disclosure and invention thus also includes a recombinant construct comprising a polynucleotide as defined herein, preferably wherein the polynucleotide is linked to a heterologous nucleic acid sequence. By "heterologous" as used herein is meant a polynucleotide sequence which is not natively linked to the polynucleotide described herein, i.e. which is not linked to the polynucleotide described herein in nature. In the construct, the polynucleotide described herein may be flanked by restriction sites (i.e. nucleotide sequences recognised by one or more restriction enzymes) to enable easy cloning of the polynucleotide of the invention. A "recombinant" construct is a polynucleotide construct synthesised using recombinant techniques, e.g. molecular cloning.

The term "linked" as used herein with respect to the construct may simply mean that the polynucleotide is directly joined to a heterologous nucleic acid sequence. In a preferred embodiment, in the recombinant construct the polynucleotide disclosed herein is operably linked to a heterologous expression control sequence, such that the expression control sequence regulates the expression of the polynucleotide. Thus, also provided is an expression cassette comprising a polynucleotide as defined herein, or a vector that comprises a polynucleotide or a recombinant construct as defined herein. Expression vectors are routinely constructed in the art of molecular biology and may for example involve the use of plasmid DNA and appropriate initiators, promoters, enhancers, terminators and other elements, such as for example polyadenylation signals which may be necessary, and which are positioned in the correct orientation, in order to allow for expression of a fusion polypeptide, a bispecific binding molecule, conjugate, or chain thereof. Other suitable vectors would be apparent to persons skilled in the art, and include e.g. cloning vectors. The invention also provides cells that comprise the polynucleotide, recombinant construct, or vector of the invention. Such a cell is a cell into which such a molecule, construct or vector has been introduced. The cell may be defined as a host cell, e.g. a cloning host cell (used within the synthesis and/or production of the polynucleotide, recombinant construct or vector) or a production host cell (used for expression of the protein(s) encoded by the polynucleotide, recombinant construct or vector).

Such cells include transient, or preferably stable, higher eukaryotic cell lines, such as mammalian cells or insect cells, lower eukaryotic cells, such as yeast or prokaryotic cells such as bacterial cells. Particular examples of cells include mammalian HEK293T, CHO, HeLa, NSO and COS cells. Preferably the cell line selected will be one which is not only stable, but also allows for mature glycosylation and cell surface expression of a polypeptide. Such cells may be cultured using routine methods to produce the fusion polypeptide or bispecific binding molecule of the invention.

In a sixth aspect of the disclosure, there is provided_a composition comprising a fusion polypeptide as disclosed herein and at least one pharmaceutically acceptable excipient or carrier. In an embodiment, there is provided a composition comprising a loaded decorated liponanoparticle as disclosed herein or a complex as disclosed herein and at least one pharmaceutically acceptable excipient or carrier.

In a seventh aspect of the disclosure, there is provided a kit comprising a loaded decorated liponanoparticle as disclosed herein, and at least one bispecific binding molecule as disclosed herein; and instructions for use of said kit. In such kits, the loaded decorated liponanoparticle and bispecific binding molecule may be separately provided in compositions comprising a pharmaceutically acceptable carrier or excipient.

As used herein, "pharmaceutically acceptable carrier or excipient" includes any and all solvents, dispersion media, coatings, antibacterial and antifungal agents, isotonic and absorption delaying agents and the like that are physiologically compatible.

Preferably, the carrier or excipient is suitable for parenteral, e.g., intradermal, intravenous, intramuscular or subcutaneous administration (e.g., by injection or infusion). Depending on the route of administration, the fusion polypeptide, loaded decorated liponanoparticle, complex or constituent component thereof may be coated in a material to protect it from the action of acids and other natural conditions that may inactivate or denature it.

Preferred pharmaceutically acceptable carriers comprise aqueous carriers or diluents. Examples of suitable aqueous carriers that may be employed in the compositions and kits of the sixth and seventh aspects, respectively, include water, buffered water and saline. Examples of other carriers include ethanol, polyols (such as glycerol, propylene glycol, polyethylene glycol, and the like), and suitable mixtures thereof, vegetable oils and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be preferable to include isotonic agents, for example, sugars, polyalcohols such as mannitol, sorbitol, sodium chloride and the like.

The composition or kit also may include a pharmaceutically acceptable antioxidant. They may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol sorbic acid, and the like. In addition, prolonged absorption of the injectable pharmaceutical form may be brought about by the inclusion of agents which delay absorption such as aluminum monostearate and gelatin.

Therapeutic compositions typically must be sterile and stable under the conditions of manufacture and storage. The composition can be formulated as a solution, microemulsion, liposome, or other ordered structure suitable to high drug concentration.

Sterile injectable solutions can be prepared by incorporating the active agent (e.g., complex) in the required amount in an appropriate solvent with one or a combination of ingredients enumerated above, as required, followed by sterilization microfiltration. Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile-filtered solution thereof.

Compositions and kits may comprise additional active ingredients as well as the fusion polypeptide, loaded decorated liponanoparticle, complex or component thereof, for example they may comprise additional therapeutic or prophylactic agents. Thus, the loaded decorated liponanoparticle or complex may be used as a monotherapy or as part of a combination therapy, e.g., in the treatment of cancer. A kit as described herein may additionally contain instructions for use.

In an eighth aspect of the disclosure, there is provided a method of producing a fusion polypeptide as disclosed herein, a loaded decorated liponanoparticle as disclosed herein, or a complex as disclosed herein. In one embodiment, there is provided a method of producing a fusion polypeptide as disclosed herein, the method comprising:
- culturing a host cell as disclosed herein under conditions allowing expression of said fusion polypeptide from said expression vector; and
- isolating said fusion polypeptide.

The fusion polypeptide as disclosed herein may alternatively be produced by non-biological peptide synthesis using amino acids and/or amino acid derivatives having protected reactive side-chains. Therein, in another embodiment there is provided a method of producing a fusion polypeptide as defined herein, the method comprising:
- step-wise coupling of the amino acids and/or the amino acid derivatives to form a polypeptide according to the first aspect having protected reactive side-chains,
- removal of the protecting groups from the reactive side-chains of the polypeptide, and thereby
- obtaining of the polypeptide in aqueous solution.

In an aspect of the present disclosure, there is provided a method of producing a loaded decorated liponanoparticle as disclosed herein, the method comprising:
- combining at least one phospholipid, and optionally at least one sterol and/or at least one lipid, with a payload as defined herein, to obtain a loaded liponanoparticle;
- providing at least one fusion polypeptide as disclosed herein; and
- combining said fusion polypeptide with said loaded liponanoparticle to obtain a decorated loaded liponanoparticle.

The decorated liponanoparticle as defined herein comprises the fusion polypeptide coupled to its surface by means of the lipid binding domain of the apolipoprotein or fragment thereof comprised in said fusion polypeptide. The liponanoparticle comprises a core which comprises the payload, thus is a loaded liponanoparticle.

In another embodiment, there is provided a method of producing a loaded decorated liponanoparticle as disclosed herein, the method comprising:
- combining at least one ionizable lipid; 1,2-distearoyl-sn-glycero-3-phosphocholine; cholesterol; at least one stabilizer, such as 1,2-Dimyristoyl-rac-glycero-3-methoxypolyethylene glycol-2000, with a payload as defined herein, to obtain at least one loaded liponanoparticle;
- providing at least one fusion polypeptide as disclosed herein; and
- combining said at least one fusion polypeptide with said at least one loaded liponanoparticle to obtain a loaded decorated liponanoparticle.

In another embodiment there is provided a method of producing a complex as disclosed herein, the method comprising:
- providing at least one bispecific binding molecule as disclosed herein,
- providing at least one loaded decorated liponanoparticle as disclosed herein; and
combining said at least one bispecific binding molecule with said at least one loaded decorated liponanoparticle, to obtain said complex.

The skilled person appreciates that the fusion polypeptide, the loaded decorated liponanoparticle, the complex, the composition as well as the kit as disclosed in first tothird and sixth to seventh aspects as described herein may be useful in therapy. In particular usefulness is anticipated in targeted delivery to a therapeutically and/or prophylactically active payload to a target tissue, organ or cell. In particular, it is desirable that a payload is delivered to the complex target tissue, organ or cell of interest. It is envisioned, without being bound by theory, that targeted delivery may allow for therapeutically effective amounts of the therapeutically and/or prophylactically active agents to be delivered while reducing the number and severity of adverse side effects compared to non-targeted delivery of the same active compound, which would require higher doses to achieve the same therapeutic and/or prophylactic effect. By therapy is meant the treatment of a subject. By "therapy" as used herein is meant the treatment of any medical condition. Such treatment may be prophylactic (i.e., preventative), curative (or treatment intended to be curative), or palliative (i.e., treatment designed merely to limit, relieve or improve the symptoms of a condition). In curative and palliative applications, complexes or compositions are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as a "therapeutically effective amount". Effective amounts for a given purpose will depend on the disease or condition to be treated, its severity and the size/weight and general state of the subject.

Prophylactic treatment may include the prevention of a condition, or a delay in the development or onset of a condition. For example, the complex may be used to prevent an infection, or to reduce the extent to which an infection may develop, or to prevent, delay or reduce the extent of a cancer developing, or recurring, or for example to prevent or reduce the extent of metastasis.

Thus in a ninth aspect of the present disclosure, there is provided a fusion polypeptide as defined herein, a loaded decorated liponanoparticle as defined herein, complex as defined herein or a kit as defined herein for use in therapy of a subject in need thereof.

As used herein, the term "subject" refers to any mammal, e.g., a farm animal such as a cow, horse, sheep, pig or goat, a pet animal such as a rabbit, cat or dog, or a primate such as a monkey, chimpanzee, gorilla or human. Most preferably the subject is a human.

The present inventors also consider a similar effect and advantage can be achieved by a combination product, which comprises the bispecific binding molecule as defined herein and loaded decorated liponanoparticle as defined herein, wherein said bispecific binding molecule and loaded decorated liponanoparticle are administered to a subject in need thereof in separately, simultaneously or sequentially, such that the complex as defined herein is formed in vivo in the patient and results in targeted delivery to the payload. Thus, in one embodiment there is provided a product, or a combination product, comprising a bispecific binding molecule as defined herein and loaded decorated liponanoparticle as defined herein as a combined preparation for separate, simultaneous or sequential use in therapy of a subject in need thereof. Similarly, when the kit of the disclosure is used in therapy, the bispecific binding molecule and loaded decorated liponanoparticle are administered simultaneously or sequentially to the subject.

By "simultaneous" administration, as used herein, means that the two components are administered to the subject at the same time, or at least substantially the same time, by the same administrative route and at substantially the same location. By "sequential" administration, as used herein, is meant that the two components are administered to the subject at different times. In particular, administration of the first component is completed before administration of the second component commences. Sequential administration as envisioned herein, requires both to be administered by the same route and at substantially the same location. Furthermore, although the administration of the bispecific binding molecule and loaded decorated liponanoparticle may be at different time points, the interval between the administrations should be such as to allow a complex to be formed, when both components have been administered. Thus, for example, both components may be administered within 1 hour of each other, or more particularly within 40, 30, 20, 15, 10, 8, 7, 6, 5, 4, 3, 2 or 1 minute, or less than a minute, of each other.

As discussed in the context of third aspect of the present disclosure, relating to the complex as disclosed herein, the identity of the first specific binding molecule of the bispecific binder dictates what target antigen said first specific binding molecule has affinity for and thus to what organ, tissue or cell the complex is targeted. By selecting the first specific binding molecule with affinity for a desired target antigen, it is possible to use the complex for delivery to any organ, tissue or cell which expresses said target antigen. In addition, by selecting the appropriate payload to be delivered to said organ, tissue or cell, a desired therapeutic and/or prophylactic effect may be achieved in said organ, tissue or cell.

Non-limiting examples of target antigens for which said least one first specific binding molecule comprised in the bispecific binding molecule are CD40, HER2, and CD20. Thus, the fusion polypeptide, loaded decorated liponanoparticle, complex, kit and/or product as defined herein may be used in the treatment or prevention of cancer. It is in particular envisioned that targeted delivery may be to desired to cancer cells, not limited to solid tumors, or to cells infected by pathogens, or to sites of inflammation. It is also envisioned that the fusion polypeptide, loaded decorated liponanoparticle, complex, composition, kit or combined product as disclosed herein may be useful in the treatment of autoimmune disease.

By cancer is meant any malignant or pre-malignant neoplastic condition. The cancer may thus be any cancer, of any organ, tissue or cell type. Cancers which present as solid tumors, and which do not exhibit solid tumors are included. Accordingly, haemopoietic cancers are included. Non-limiting examples of cancers are prostate cancer, breast cancer, colorectal cancer, pancreatic cancer, ovarian cancer, lung cancer, cervical cancer, rhabdomyosarcoma, neuroblastoma, multiple myeloma, leukemia, acute lymphoblastic leukemia, melanoma, bladder cancer, head and neck cancer, lymphoma, glioblastoma, and skin cancer. It may also be adrenal cancer, bone cancer, brain cancer, esophageal cancer, eye cancer, gastric cancer, oral cancer, penile cancer, testicular cancer, thyroid cancer, uterine cancer, and vaginal cancer. Mast cell tumors and hemangiosarcoma may also be treated according to the present disclosure. The cancer may be newly diagnosed and naïve to treatment or it may be relapsed or refractory, or relapsed and refractory, primary or metastatic.

In the case wherein said first binding molecule of the bispecific binding molecule has affinity for CD40, it is envisioned that the first CD-40 binding molecule activates the immune system by agonizing or stimulating CD40 on APCs, particularly on dendritic cells. In particular, this may lead to T cell activation. The subsequent immune response exerts an anti-cancer effect on neighboring or accessible tumor cells, without regard to CD40 expression by the tumor. Thus, the complex as defined herein may therefore be effective against both CD40-positive and CD40-negative cancers. For CD40-positive cancers, it is envisioned that the payload comprised in the loaded decorated liponanoparticle may have for example cytotoxic effect or other anti-cancer effect and by virtue of the said first binding molecule of the bispecific binding molecule has affinity for CD40 be delivered to CD40-positive tumors in a targeted manner. By a similar principle, the delivery of the payload comprised in the loaded decorated liponanoparticle with for example cytotoxic effect or other anti-cancer effect, may be achieved to HER2-positive tumors or to CD20-positive tumors.

The skilled person appreciates that for example a complex as described herein, wherein said first specific binding molecule of the bispecific binding molecule has affinity for CD40 may also exert its effect as an adjuvant within a vaccination regimen against a pathogen. In this case the loaded decorated liponanoparticle may comprise the antigen of interest in form of a DNA/RNA-based vaccine and CD40 activation may aid stimulate effective anti-pathogen immune responses resulting in neutralizing antibodies or T cell responses. The skilled person appreciates that the first specific binding molecule of the bispecific binding molecule may be selected to target a disease on interest and the payload may be selected to exert a desired therapeutic and/or prophylactic effect. Thus the fusion polypeptide, loaded decorated liponanoparticle, complex, composition, kit or combined product as disclosed herein may be useful as a vaccine and also may be useful in the treatment or prevention of an infection, such as a viral infection or a bacterial infection.

In a tenth related aspect there is provided a method of treatment of a disease, disorder or infection, comprising administering to a subject in need thereof of a therapeutically effective amount of a fusion polypeptide as defined herein, a loaded decorated liponanoparticle as defined herein, a complex as defined herein, or a pharmaceutical composition as defined herein. It is contemplated that relevant diseases, disorders or infections could be selected from the group comprising cancer, infectious diseases, brain disorders, inflammatory diseases, genetic disorders, and vascular diseases. Said method may be at therapeutic treatment method and/or prophylactic treatment method.

In a related eleventh related aspect there is provided use of a fusion polypeptide as defined herein, a loaded decorated liponanoparticle as defined herein, a complex as defined herein, a composition as defined herein, or a kit as defined herein in the manufacture of a medicament.

The skilled person appreciates that the embodiments and reasoning discussed in the context of the ninth aspect of the present disclosure are equally relevant for the tenth and eleventh aspect and are not repeated here merely for the sake of brevity.

It is also envisioned that the loaded decorated liponanoparticle, complex, composition, kit or combined product as disclosed herein may be useful as diagnostic and/or progronostic agent. In such embodiments, said loaded decorated liponanoparticle may contain a payload which binds to a marker of interest, whereby the presence or absence or quantity of said marker may be scored, directly or indirectly, for example, but not limited to, by medical imaging. For example, said payload may contain a radionuclide label or a fluorescent label or any other suitable label. Thus, in a twelfth aspect there is provided a fusion polypeptide as defined herein, a loaded decorated liponanoparticle as defined herein, a complex as defined herein, a pharmaceutical composition as defined herein, or a kit as defined herein for use as a diagnostic agent *in vitro,* for use as a diagnostic agent *in vivo,* for use as a prognostic agent *in vitro,* for use as a prognostic agent *in vivo.* In one embodiment, there is provided a fusion polypeptide, a loaded decorated lipidnanoparticle, a complex, a composition, or a kit for use as defined herein as diagnostic agent in the *in vitro* or *in vivo* diagnosis of a disease, disorder and/or infection. In another embodiment, there is provided a fusion polypeptide, a loaded decorated liponanoparticle, a complex, a composition, or a kit for use as defined herein as a prognostic agent in the *in vitro* or *in vivo* prognosis of a disease, disorder and/or infection.

In a related thirteenth aspect, is also provided is a method for determining the presence of a marker, such as a marker indicating a disease, disorder and/or infection, in a subject or in a sample isolated from said subject, comprising the steps of:
a) contacting said subject or said sample isolated from said subject, with a loaded decorated liponanoparticle as defined herein, a complex as defined herein or a composition as defined herein; and
b) obtaining a value corresponding to the amount of loaded decorated liponanoparticle, complex or composition that has bound in said subject or to said sample.

In a yet another related aspect there is provided a method of diagnosis or prognosis, comprising the steps of:
a) contacting a subject or a sample isolated from said subject, with a loaded decorated liponanoparticle as defined herein, a complex as defined herein, a composition as defined herein; and
b) obtaining a value corresponding to the amount of loaded decorated liponanoparticle, complex or composition that has bound in said subject or to said sample.

In one embodiment, said method of diagnosis or prognosis is a method for diagnosis *in vivo* or a method for prognosis *in vivo,* for example via medical imaging, in which said contacting in step a) consists of contacting said subject with said loaded decorated liponanoparticle, complex or composition. In one embodiment, said method of diagnosis or prognosis is a method for diagnosis *in vitro* or method for prognosis *in vitro,* in which said contacting in step a) consists of contacting a sample previously isolated from said subject with said loaded decorated liponanoparticle, complex or composition. In one embodiment, said subject is a mammalian subject, such as a human subject.

As explained in the context of the present disclosure, the loaded decorated liponanoparticle, the complex and the composition as defined herein are useful in the delivery of a payload. Thus, in this fifteenth aspect, there is provided an *in vivo* or *in vitro* method for introducing a nucleic acid or a nucleic acid analog, such as RNA, DNA or nucleic acid analog as defined herein, into at least one cell, the method comprising:
contacting the loaded decorated liponanoparticle as defined herein or the composition as defined herein, with said at least one cell.

In addition, if targeted delivery is desired, the complex as defined herein may be utilized. Thus, there is provide an *in vivo* or *in vitro* method for introducing a nucleic acid or a nucleic acid analog, such as RNA, DNA or nucleic acid analog as defined herein into at least one cell, the method comprising:
contacting the complex comprising a bispecific binding molecule and a loaded decorated liponanoparticle as defined herein with said at least one cell, wherein said at least one cell expresses the target antigen for which said at least one first specific binding molecule of said bispecific binding molecule has affinity.

### Examples

The following examples disclose the development of at least one fusion polypeptide comprising a non-immunogenic tag moiety sequence and a polypeptide comprising an apolipoprotein or fragment thereof. The generic amino acid sequence of said tag moiety described herein is listed in the sequence listing with SEQ ID NO:1. Said fusion polypeptide may be displayed on the surface layer of a loaded decorated lipidnanoparticle (LNP). Therein, the examples further describe the characterization of at least one loaded decorated LNP comprising a core surrounded by a surface layer, wherein said core comprises at least one payload and said surface layer comprises at least one phospholipid and said at least one fusion polypeptide. The examples further describe the characterization of complexes comprising said at least one loaded decorated LNP, and demonstrate their *in vitro* and *in vivo* functionality.

### Example 1

### Binding affinity between tag moiety and second specific binding molecule

The binding affinity of various tag moieties, with various C-terminal modifications, to a bispecific binding molecule comprising a second specific binding molecule (SEQ ID NO:436) with affinity for said tag moiety was evaluated.

### Materials and Methods

To evaluate if spacer elements and cargo peptide sequences (SEQ ID NO:271-278) introduced C-terminally to any of the tag moiety sequences (SEQ ID: 2-4) impacted binding to a bispecific binding molecule, said bispecific binding molecule comprising a first specific binding molecule (comprising SEQ ID: 265 and266) designed to target CD40 and a second specific binding molecule (SEQ ID NO:436) designed to bind any tag moiety sequence (SEQ ID:2-7), and variants thereof, a competition assay, fluorescence polarization (FP) assay, was run. The displacement (equilibrium phase that includes labeled and unlabeled peptide) was performed in 25mM Histidine buffer, pH 6. The measurements were performed on a SpectraMax iD5 plate reader (Molecular Devices) at room temperature and at excitation/emission wavelengths of 485/535 nm.

The FP assay utilized a tag moiety sequence (SEQ ID NO:2) which was fluorescently labelled with AlexaFluor 488 (AF488) (SEQ ID NO:448) via maleimide-thiol conjugation of AF488 to the an additional Cys residue located C-terminally on the tag moiety, to evaluate displacement of various unlabeled peptides. The unlabeled peptides comprised a tag moiety sequence (SEQ ID NO:2-4) with or without spacer elements based on AAA, AAY and GGS sequences and a cargo peptide sequence (SEQ ID NO:228-245). All peptides utilized in the FP Assay were produced by solid phase chemistry (synthetic production). The FP assay included binding analysis by measuring fluorescence and subsequent curve fitting and normalizing the resulting data to a reference data point of a known peptide sequence comprising a tag moiety sequence (SEQ ID NO:2), to which the binding affinity to a bispecific binding molecule had previously been evaluated (data not shown) using surface plasmon resonance (SPR) analysis.

### Results

The binding analysis data for the tested peptides is presented below in Table 1 with the calculated affinities:

**Table 1: Calculated affinities of various tag moieties, with or without various C-terminal modifications, to a bispecific binding molecule**

| **SEQ ID NO:** | **Affinity (pM)** | **Sequence** (tag moiety sequence in **BOLD,** followed by a spacer element if present in underlined, followed by a cargo peptide sequence if present) |
|---|---|---|
| SEQ ID NO:228 | 7,8 | **FIGITELKK**AAYRAHYNIVTFAAYRAHYNIVTFAAYRAHYNIVTF |
| SEQ ID NO:229 | 12 | **FIGITELKK**AAARAHYNIVTFAAARAHYNIVTFAAARAHYNIVTF |
| SEQ ID NO:230 | 16 | **FIGITELKK**GGSRAHYNIVTFGGSRAHYNIVTFGGSRAHYNIVTF |
| SEQ ID NO:231 | nb | **GITELKK**AAYRAHYNIVTFAAYRAHYNIVTFAAYRAHYNIVTF |
| SEQ ID NO:232 | nb | **GITELKK**AAARAHYNIVTFAAARAHYNIVTFAAARAHYNIVTF |
| SEQ ID NO:233 | nb | **GITELKK**GGSRAHYNIVTFGGSRAHYNIVTFGGSRAHYNIVTF |
| SEQ ID NO:234 | 17 | **FIGITELKK**AAYRAHYNIVTFAAYRAHYNIVTF |
| SEQ ID NO:235 | 11 | **FIGITELKK**AAARAHYNIVTFAAARAHYNIVTF |
| SEQ ID NO:236 | 12 | **FIGITELKK**GGSRAHYNIVTFGGSRAHYNIVTF |
| SEQ ID NO:237 | 76 | **FIGITELKK**GGSAVGALKVPRNQDWLGVPRQL |
| SEQ ID NO:238 | 171 | **FIGITELLK**GGSAVGALKVPRNQDWLGVPRQL |
| SEQ ID NO:239 | 63 | **FIGITELHK**GGSAVGALKVPRNQDWLGVPRQL |
| SEQ ID NO:240 | 5,5 | **FIGITELKK**SSAESLKISQAVHAAHAEINEAGREVVGSAE |
| SEQ ID NO:241 | 13 | **FIGITELLK**SSAESLKISQAVHAAHAEINEAGREVVGSAE |
| SEQ ID NO:242 | 19 | **FIGITELHK**SSAESLKISQAVHAAHAEINEAGREVVGSAE |
| SEQ ID NO:243 | 2,7 | **FIGITELKK**GGSSSAESLKISQAVHAAHAEINEAGREVVGSAE |
| SEQ ID NO:244 | 19 | **FIGITELLK**GGSSSAESLKISQAVHAAHAEINEAGREVVGSAE |
| SEQ ID NO:245 | 5,4 | **FIGITELHK**GGSSSAESLKISQAVHAAHAEINEAGREVVGSAE |
| SEQ ID NO:2 | 9,4 | **FIGITELKK** |
| SEQ ID NO:3 | 63 | **FIGITELLK** |
| SEQ ID NO:4 | 15 | **FIGITELHK** |

The calculated affinity in pM is stated in Table 1, and nb means no binding. The data shows that the introduction of spacer elements "GGS", "AAA" or "AAY" C-terminally to a tag moiety sequence does not impact affinity between a tag moiety sequence and a bispecific binding molecule comprising a second specific binding molecule (SEQ ID NO:436) with affinity for said tag moiety. This is regardless of which cargo peptide sequences (SEQ ID NO:271-278) are attached C-terminally to said tag moiety sequence, with or without a spacer element present between the tag moiety sequence and the cargo peptide sequence. N-terminal trimming of the tag moiety sequence with two amino acids (SEQ ID NO:231-233) disrupts binding to the bispecific binding molecule.

### Example 2

### Evaluation of fusion polypeptide production and binding affinity to a second specific binding molecule

This example displays the feasibility of producing a fusion polypeptide comprising a tag moiety and a polypeptide comprising an apolipoprotein or fragment thereof using recombinant protein production. The Example further confirms binding of the recombinantly produced fusion polypeptide to the second specific binding molecule (selected from SEQ ID NO:436-441).

### Materials and Methods

Design, production and binding of loaded decorated liponanoparticles (LNP) and bispecific binding molecules, as described herein, are designed, produced and binding affinity is tested. The surface layer of the loaded decorated LNP comprises at least one fusion polypeptide to which a second specific binding molecule (SEQ ID NO:436-441) can bind, wherein the second specific binding molecule is comprised in a bispecific binding molecule which in turn further comprises a first specific binding molecule with affinity for a target antigen, such as CD40, Her2 or CD20. The first specific binding molecule may be an anti-CD40 antibody comprising SEQ ID NO:265 and 266 (which comprise CDR regions as defined by SEQ ID NO:259-261 and SEQ ID NO:262-264, respectively); an anti-Her2 antibody comprising SEQ ID NO:267 and 268; or an anti-CD20 antibody comprising SEQ ID NO:269 and 270.

Recombinant fusion polypeptides encompassing full length or truncated ApoE2 proteins (SEQ ID NO: 18-22, 283, 286) are produced by fusion of a tag moiety (SEQ ID NO: 2-7), linking the N-terminus of full length or truncated ApoE to the C-terminus of a tag moiety leaving the tag moiety with a free N-terminus. The recombinant polypeptides are produced with and without linkers, such as utilizing linkers according to SEQ ID NO:9-14, or utilizing amino acid spacers such as "GGS", "AAY", or "AAA", between the apolipoprotein and the tag moiety for binding accessibility and cleavage investigations. The fusion polypeptides are produced in *E. coli* production system by transient production. Examples of fusion polypeptides with and without linkers are SEQ ID NO:33-62, 74-103, 114-144, 156-185, 197-226, 297-326 and 331-384.

Variants of tag moieties include, but are not limited to, FIGITELKK (SEQ ID NO:2), FIGITELLK (SEQ ID NO:3) and FIGITELHK (SEQ ID NO:4), with or without a linker or spacer sequence between the C-terminus of the tag moiety and the N-terminus of the unmodified or modified ApoE2 protein or fragments thereof (examples included in SEQ ID NO: 18-22, 283, and 286). The fusion polypeptide is modified to enhance the binding of the second specific binding molecule, as well as reducing the affinity for the LDL-R and the polysaccharide heparan sulphate, for a more selective binding to the second specific binding molecule. The ApoE modifications can be either unique or be a combination of different modifications, like single amino acid substitutions or truncated proteins. Five regions within ApoE2 can be modified to enhance fusion polypeptide interaction with scFv element (also referred to as the second specific binding molecule) of the bispecific binding molecule. Three regions, with an open protein structure, are suitable for truncation:
[1] KVEQAVETEPEP position 1-12 (SEQ ID NO:385),
[2] TLSEQVQEELLSSQ position 42-55 (SEQ ID NO:386), and
[3] YQAGAREGAERGLSAIRERLGPLVEQGRVRAATVGSLAGQ (SEQ ID NO:388) position 162-201 in the ApoE protein. Further, two regions important for ApoEs interaction with its receptor can be modified, [1] amino acid R61, and [2] ELRVRLASHLRKLRKRLLRDADDLQKC (SEQ ID NO:387) position 132-158 in the ApoE2 protein, modifications like R136S, H140A, K143A, L144P and R150A all reduce binding to LDL-R. Examples of the truncations and aa substitutions can be seen in SEQ ID NO: 18-22, 286.

**Table 2: Examples of fusion polypeptide constructs. SEQ ID NO is provided for the full-length fusion polypeptide construct. SEQ ID NO is also provided for the components comprised in each full-length fusion polypeptide construct: the tag moiety, the ApoE2 protein or fragment thereof, and optionally a linker.**

| **SEQ ID NO for full-length fusion polypeptide construct** | **Components comprised in the full-length fusion polypeptide construct** | | |
|---|---|---|---|
| | **SEQ ID NO for tag moiety** | **SEQ ID NO for linker** | **SEQ ID NO for ApoE protein or fragment thereof** |
| 34 | 2 | 9 | 18 |
| 75 | 2 | 9 | 19 |
| 37 | 2 | 12 | 18 |
| 78 | 2 | 12 | 19 |
| 331 | 2 | 328 | 18 |
| 335 | 2 | 328 | 19 |
| 33 | 2 | n/a* | 18 |
| 74 | 2 | n/a* | 19 |
| 44 | 3 | 9 | 18 |
| 85 | 3 | 9 | 19 |
| 47 | 3 | 12 | 18 |
| 88 | 3 | 12 | 19 |
| 349 | 3 | 328 | 18 |
| 352 | 3 | 328 | 19 |
| 43 | 3 | n/a* | 18 |
| 84 | 3 | n/a* | 19 |
| 54 | 4 | 9 | 18 |
| 95 | 4 | 9 | 19 |
| 57 | 4 | 12 | 18 |
| 98 | 4 | 12 | 19 |
| 367 | 4 | 328 | 18 |
| 370 | 4 | 328 | 19 |
| 53 | 4 | n/a* | 18 |
| 94 | 4 | n/a* | 19 |

| | | | |
|---|---|---|---|
| **fusion polypeptide construct does not comprise a linker sequence between the tag moiety sequence and the ApoE protein sequence or fragment thereof* | | | |

Fusion polypeptides presented in Table 2 are produced by bacterial or cell-based production as recombinant proteins. Alternatively, the production of the ApoE2 protein will be bacterial or cell-based and a subsequent site-specific conjugation step will be included to conjugate a synthetically produced tag moiety that includes a reactive group to the ApoE2 protein or fragment thereof.

Protein expression is verified using protein concentration measurements using standard methodologies in the lab of the producer.

The designed and produced fusion polypeptides bind to bispecific binding molecules which are designed and produced to comprise a second specific binding molecule (SEQ ID NO:436-441) with affinity for the tag moiety comprised in the fusion polypeptide. Data is presented in a table and can include binding data from fusion polypeptides, for example fusion polypeptides selected from SEQ ID NO: 33-62, 74-103, 115-144, 156-185, 197-226, 297-326 and 331-384. The experiment may also include ApoE2 proteins or fragments thereof not comprising a tag moiety sequence, such as selected from SEQ ID NO:18-22, 283, and 286, which should not bind to a second specific binding molecule. Appropriate methodologies may include surface plasmon resonance or alternative binding methods such as blot techniques, fluorescent polarization techniques or ELISA based technologies by immobilizing the bispecific binding molecule (such as a bispecific binding molecule comprising a second specific binding molecule according to sequences selected from SEQ ID NO: 436-441 and a first specific binding molecule comprising sequences SEQ ID NO:265 and 266; SEQ ID NO:267 and 268; or SEQ ID NO:269-270) on the surface and adding the recombinantly produced fusion polypeptide and subsequently detect binding utilizing the various techniques mentioned above. For ELISA this will also include a step of identification using an anti-ApoE secondary antibody (ELISA) for detection. Affinity measurements will be performed by titration experiments and should be evaluated to be in similar range (low nM to pM range) as synthetically made fusion polypeptides that comprise a tag moiety sequence. Recombinant proteins are also analyzed in terms of quality (monomeric fraction using HPLC analysis) with regards to monomeric content and using western blot to confirm size.

### Results

The results are expected to show successful production of fusion polypeptides comprising an apolipoprotein or fragment thereof and a tag moiety. Furthermore, the results are expected to show that the produced fusion polypeptides successfully bind a bispecific binding molecule. The results are expected to show some differences in binding to the bispecific binding molecule when testing the various fusion polypeptides, various linker sequences or the lack of linker sequence, and various tag moieties.

### Example 3

### Generation of complexes comprising loaded decorated liponanoparticle (LNP)

This example displays the feasibility of generating loaded decorated liponanoparticles (LNPs) with or without a lipophilic dye, wherein the surface layer of the loaded decorated LNP comprises at least one of the produced fusion polypeptides from SEQ ID NO: 33-62, 74-103, 115-144, 156-185, 197-226, 297-326 and 331-384, and wherein the core of the loaded decorated LNP comprises a payload, such as mRNA encoding various fluorescent or luminescent reporter constructs (SEQ ID NO:279-282).

### Materials and Methods

Loaded Liponanoparticles (LNPs) are assembled using microfluidics. A lipid mixture comprising: cationic, structural/helper and stabilizer lipids are combined with anionic nucleic acids in an acidic formulation solution and a neutral storage buffer using three different flow channels. The lipid mixture used is GenVoy ionizable lipid mixture (ionizable lipid 50 mol %, DSPC 10 mol %, Cholesterol 37.5 mol %, Stabilizer(DMG-PEG 2000) 2.5 mol %). Electrostatic interactions between the cationic lipids and the anionic nucleic acid facilitates assembly of the loaded LNPs. Particle uniformity is determined by N to P ratio, the ratio between amino and phosphate groups on the cationic lipids and backbone of the nucleic acid, respectively. Generated loaded LNPs are analyzed for polydispersity using dynamic light scattering and nucleic acid incorporation determined using fluorescent dye based assays.

The data aim to show proper assembly of microfluidics generated loaded LNPs, meaning even particle size distribution, determined by the polydispersity index ≤0.2 and successful incorporation of the mRNA payload is ≥80%. Loaded LNPs are contacted with wtApoE and/or fusion polypeptide variants according to the present invention as described herein. Generated loaded decorated LNPs also comprise on their outer surface layer wild type ApoE and/or fusion polypeptide variants, from Example 2. Comparative evaluation of the success of fusion polypeptide incorporation and/or wt ApoE incorporation into the surface layer of the loaded decorated LNP is evaluated in four different assays:
1. DLS/Nanosight/Size exclusion chromatography to determine size of the loaded decorated liponanoparticle-bispecific binding molecule (the complex).
2. By fluorescent quantification nanosight analysis wherein the complex (comprising loaded decorated liponanoparticle and bispecific binding molecule) is linked to a fluorescently conjugated antibody enabling quantification of properly assembled complexes.
3. Differential expression of mRNA encoded reporter constructs from Example 4 (SEQ ID NO:279-282), following preincubation with fusion polypeptides listed in Example 2, wherein the fusion polypeptide comprises wild type ApoE protein or an ApoE variant or fragment thereof (SEQ ID: 33-62, 74-103, 115-144, 156-185, 197-226, 297-326 and 331-384). Here fusion polypeptides comprising wild type ApoE show higher and expression while fusion polypeptides comprising truncated ApoE proteins show reduced construct expression since LDL-R mediated uptake has been reduced or deleted in the ApoE variants.
4. Differential expression of reporter constructs in an immortalized cell line expressing target protein, like CD40. Here, loaded decorated LNP particles comprising a surface layer comprising fusion polypeptides, wherein said fusion polypeptide comprises wild type ApoE, show reduced uptake and expression compared to loaded decorated LNP particles comprising a surface layer comprising fusion polypeptides, (SEQ ID NO: 33-62, 74-103, 115-144, 156-185, 197-226, 297-326 and 331-384) bound to bispecific binding molecule. The assembled complex comprising loaded decorated LNP, comprising a surface layer comprising fusion polypeptides, and bispecific binding molecule, targets CD40 proteins expressed on the cell lines, mediating higher uptake and expression.

The data aim to show loaded decorated LNPs displaying on their surface layer fusion polypeptide (such as SEQ ID NO: 33-62, 74-103, 115-144, 156-185, 197-226, 297-326 and 331-384) bound with a bispecific binding molecule comprising a first specific binding molecule (targeting for example CD40, HER2 or CD20) forms a homing complex. Assessment of the complexes, will include testing complexes comprising various bispecific binding molecules, comprising a first specific binding molecule (for example an anti-CD40 antibody comprising SEQ ID NO:265 and 266; an anti-Her2 antibody comprising SEQ ID NO:267 and 268; or an anti-CD20 antibody comprising SEQ ID NO:269 and 270) and a second specific binding molecule (for example comprising a sequences selected from SEQ ID NO:246-251 and sequence SEQ ID NO: 255) and said fusion polypeptides (such as SEQ ID NO: 33-62, 74-103, 115-144, 156-185, 197-226, 297-326 and 331-384), to optimize composition of the complex (size, homing, and expression). Methodologies for testing the various complexes could be utilizing SEC followed by analysis on DLS and/or Nanosight instruments.

### Results

The results from Example 3 are expected to show successful production of loaded decorated liponanoparticles comprising fusion polypeptide bound to bispecific binding molecule as discussed above using the fusion polypeptide constructs from Example 2 (SEQ ID: 33-62, 74-103, 115-144, 156-185, 197-226, 297-326 and 331-384). The different complexes are expected to be different in size because the complexes will comprise fusion polypeptides comprising different truncated ApoE proteins, used to assemble the loaded decorated LNPs. Data are also expected to show that by varying the parts comprised in the fusion polypeptide (for example, varying linker sequence, tag moiety sequence, and apolipoprotein or fragment thereof sequence), said fusion polypeptides are expected to bind bispecific binding molecules targeting, for example, CD40, HER2 or another targets, more or less efficiently. Results will demonstrate optimal assembly conditions of the complexes.

### Example 4

### CD40 targeting of complexes

This example shows preferential uptake in cells expressing CD40 when targeted by complexes comprising loaded decorated liponanoparticle and bispecific binding molecule with affinity for CD40 conjugated to Alexa Flour 488. The complex includes CD40 targeting bispecific binding molecule (for example an anti-CD40 antibody comprising SEQ ID NO:265 and 266) and a second specific binding molecule (for example comprising sequences selected from SEQ ID NO:246-251 and sequence SEQ ID NO: 255)). Following administration, intracellular bispecific binding molecule and DIL dye labeled loaded decorated LNP uptake can be determined by fluorescent emission of the two different fluorescent dyes. The uptake is evaluated in:
1. Isolated primary B cells using in vitro cultures to show antibody-targeted uptake of loaded decorated LNPs comprising fusion polypeptide.
2. Mixed cell system, Cell expressing CD40 together with cells with no expression of CD40, with matched HLA haplotypes to avoid rejection reactions.
3. Mixed cell system, Cell expressing high levels of CD40 together with cells expressing moderate levels of CD40.

### Materials and Methods

From donated blood, immune cells are isolated using ficoll-based density gradient separation. Cells are counted and target cell types (i.e B cells, T cells, monocytes, dendritic cells) isolated using negative selection. Cells are seeded in mono or co-culture and the complex is added to mixed or monoculture system. Uptake is determined by fluorescence from the directly conjugated antibody or loaded decorated LNP incorporated dye.

Analysis of uptake of the fluorescently conjugated complex is done using flow cytometry or microplate assays. An example could be collecting monoculture and mixed culture cells at various timepoints following administration of the inventive composition (for example: 0, 5, 15, 30 min, 1h, 2h, 4h, 8h, 24h) followed by fluorescence intensity analysis to determine uptake. As a control a fluorescently conjugated complex comprising a bispecific binding molecule specific for a target not present in the donated blood is utilized. For example, a fluorescently conjugated complex wherein the first specific binding molecule is an anti HER2 antibody comprising SEQ ID NO:267 and 268, will be used to compare targeted uptake, as B cells do not express HER2. B cells are cultured utilizing RPMI with 10% FCS and 50 units/ml Penicillin & Streptomycin. Primary B cells are kept for 24 -48 hours in culture. T cells are cultured utilizing RPMI 1640 with 10% human serum, supplemented with L-Arginine, Glutamax, HEPES and 50IU of recombinant IL-2.

### Results

The results from Example 4 are expected to show specific uptake of loaded decorated LNPs in targeted cells expressing CD40 measured by fluorescence specific for loaded decorated LNPs and anti-CD40 fluorescently conjugated bispecific binding molecule being detected intracellularly in CD40 expressing cells. We expect higher uptake in cells expressing target (CD40) in mixed co-culture. We expect results to show differences in uptake rate, and expression of reporter targets in co-cultures of cells with high or low expression of CD40 which mediates uptake of the complexes. It is expected that no specific uptake of the loaded decorated LNPs is observed for the control complex. The example can also be performed using cells expressing for example CD20 or HER2 if using fluorescently labelled bispecific binding molecule targeting these proteins and the respective control complex does not comprise a bispecific binding molecule with affinity therefor.

### Example 5

### Targeted delivery and translation of mRNA

This example displays the delivery and subsequent production of a reporter construct encoded by an mRNA sequence comprised in the complex. The reporter construct mRNA include nanoluciferase (SEQ ID NO:279), firefly luciferase (SEQ ID NO:280), EGFP (SEQ ID NO:281), or gaussia luciferase (SEQ ID NO:282). Construct mRNA encoding reporters is comprised in the core of a loaded decorated LNP using the microfluidic system described in Example 3 and then incorporated in the complex.

### Materials and Methods

High CD40 expressing immortalized cell line (B cell lymphoma cell lines: for example, EHEB, RC-K8, OCI-Ly8, OCI-Ly3) will be used to study expression of reporter constructs.

Expression of reporter construct protein will be analyzed using flow cytometry, ELISA, plate based assays, western blot and/or RT-qPCR depending on the study aim of the specific experiment (for example: mRNA to protein translation efficiency, endosomal escape etc.). Experiments will evaluate the different fusion polypeptides comprising truncated ApoE variants, tag moiety with or without linkers to assess if the different complexes described above affect mRNA delivery or translation.

### Results

The results of Example 5 are expected to show the feasibility of targeted delivery of and translation of mRNA in comparison to cells exposed to dummy coated antibodies, antibodies with a binding specificity other than CD40, that cannot specifically target and bind CD40 on the cell surface. We expect the data will show reporter construct translation advantages/disadvantages of various fusion polypeptides comprising various tag moiety sequences, linker, ApoE combinations (SEQ ID NO: 33-62, 74-103, 114-144, 156-185, 197-226, 297-326 and 331-384). Additionally we expect the results to show that different tag moiety sequences (SEQ ID NO: 2-7) could show improved mRNA delivery or endosomal escape. Similar to example 3, loaded decorated LNPs will be compared to loaded LNPs wherein the LNP comprises wildtype ApoE protein to evaluate the translational efficiency of the complexes comprising fusion polypeptide according to the invention.

### Example 6

### In vivo delivery of loaded decorated LNPs with bispecific binding molecule

This example shows *in vivo* delivery to the lymph nodes and spleen after IV injection of a loaded decorated LNP without a bispecific binding molecule, a loaded decorated LNP with a bispecific binding molecule that cannot target a cell *in vivo* and a loaded decorated LNP with a bispecific binding molecule that can target a specific cell type in the body.

### Materials and Methods

Production of protein from delivered mRNA from cells isolated from spleen and lymph nodes will be compared between animals to analyze and verify targeted delivery of loaded decorated LNPs and compare various tags and linkers for optimized construct design. Cells from the spleen and the lymph node can be processed mechanically and via filters to single cells. The protein production can be analyzed both by using imaging (when using luciferase as a reporter gene) or using non-endogenous proteins and measuring protein content after cell processing using western blot from cell lysate. Alternatively ELISA will be used to detect the translated protein from protein lysase. In addition, eptiopes presented from the protein will be analyzed by studying specific T cell expansion and/or T cell responses (by ELISpot or flow cytometry activation panels) in response to immunogenic epitopes such as gp100, ovalbumin derived epitopes, viral derived antigens such as the E7 immunodominant epitope along with defined neoantigens from the MC38 colorectal tumor.

Specifically mice that have the expression of the targeted protein of interest, for example human CD40, will be used. The human CD40 protein is then expressed on antigen-presenting cells such as dendritic cells, macrophages and B cells. A designed anti-CD40 bispecific binding molecule will be preloaded prior injection with various ratios of loaded decorated LNPs. Controls are loaded decorated LNP without a bispecific binding molecule as well as loaded decorated LNP with a bispecific binding molecule that cannot target a cell *in vivo* in the mouse model. For each experimental condition around 4-6 mice will be studied per experiment. Therapy can be given 1, 2 or 3 times and both by subcutaneous and intravenous injection. It can be 3-7 days apart between each injection. Studies of protein expression and pharmacodynamic responses will then take place 4, 24, 48 or 72 hours post an injection.

### Results

The results of Example 6 are expected to show verification of cell targeting *in vivo* along with protein production from the delivered mRNA along with comparison data of LNPs comprising fusion polypeptides wherein tag moiety, linkers and/or ApoE protein or fragments thereof are varied in order to determine optimized intracellular routing of the LNP and their respective payload, such as mRNA. Data will confirm that targeted delivery can provide genetic correction and substituting protein expression by delivery of a coding element as a means to substantiate the broader applicability of the platform.

The data will also include confirmation of the therapeutic impact of the proteins produced by the delivered mRNA using T cell expansion as a readout, or alternatively using ELISpot or flow cytometry based readout to confirm T cell activation.

### Example 7

### Improved effect of targeted delivery mRNA using a bispecific binding molecule

This example shows *in vivo* impact of targeted delivery of mRNA using a complex compared to loaded decorated LNP without a bispecific binding molecule as well as loaded decorated LNP with a bispecific binding molecule that cannot target a cell *in vivo* in the mouse model.wherein said payload comprises mRNA derived from one or several known immunogenic neoepitopes derived from the MC38L model. Animals growing MC38L colorectal tumors are treated with the complex or with control via IV and/or SC administration route. The best construct(s) from Example 6 will be used to verify *in vivo* effective tumor induced killing by immune cell activation.

### Methods and Materials

Mice (18-20 gram) will be injected with 300 000 MC38L cells. After 5-7 days treatment will be initiated. Treatment will be administrated with various doses of the formulated complex or control will be assessed, and the complex or control(s) will be administered to test subjects by either intravenous or subcutaneous administration. Ratios of loaded decorated LNP to bispecific binding molecule along with repeated dosing (day 6, 10 and 14 as an example) will be assessed. Tumor size will be measured using a caliper and survival will be monitored. Humane endpoint will include 1000 mm3 of tumor volume along with wounds and weight of mice.

### Results

The results are expected to demonstrate improved effect of targeted delivery to a specific cell of interest, compared to loaded decorated LNPs that are not targeted to that specific cell type, using anti-tumor responses as a means to evaluate such response. Verification of improved anti-tumor response by the target delivery achieved by the inventive concept compared to control.

### Itemized list of embodiments

1. Fusion polypeptide comprising
   i) a tag moiety comprising or consisting of the amino acid sequence
      FIGITELX₈X₉ (SEQ ID NO:1)
      wherein
      X₈ is selected from K, L, and H; and
      X₉ is K or absent; and
   ii) a polypeptide comprising an apolipoprotein or a fragment thereof, said tag moiety is arranged N-terminally of said apolipoprotein or fragment thereof and wherein said fusion polypeptide optionally comprises a linker sequence arranged between said tag moiety and said apolipoprotein or fragment thereof.
2. Fusion polypeptide according to item 1, wherein X₈ in SEQ ID NO:1 is selected from K and L.
3. Fusion polypeptide according to item 1, wherein X₈ in SEQ ID NO:1 is selected from L and H.
4. Fusion polypeptide according to item 1, wherein X₈ in SEQ ID NO:1 is selected from K and H.
5. Fusion polypeptide according to any one of items 2 and 4, wherein in SEQ ID NO:1 X₈ is K.
6. Fusion polypeptide according to any one of items 2-3, wherein X₈ in SEQ ID NO:1 is L.
7. Fusion polypeptide according to any one of items 3-4, wherein X₈ in SEQ ID NO:1 is H.
8. Fusion polypeptide according to any preceding item, wherein X₉ in SEQ ID NO:1 is K.
9. Fusion polypeptide according to any one of items 1-7, wherein X₉ in SEQ ID NO:1 is absent.
10. Fusion polypeptide according to any preceding item, wherein said tag moiety comprises or consists of an amino acid sequence selected from the group consisting of SEQ ID NO:2-7
11. Fusion polypeptide according to any one of items 1-8 and 10, wherein said tag moiety comprises or consists of an amino acid sequence selected form the group consisting of SEQ ID NO:2-4.
12. Fusion polypeptide according to any one of items 1-8 and 10-11, wherein said tag moiety comprises or consists of the amino acid sequence SEQ ID NO:3.
13. Fusion polypeptide according to any one of items 1- 12, wherein the C terminus of said tag moiety is covalently bound to the N terminus of said apolipoprotein or to a fragment thereof.
14. Fusion peptide according to any one of items 1-13, wherein said linker is a flexible linker, a rigid linker or a cleavable linker, such as a flexible linker.
15. Fusion polypeptide according to any one of items 1-14, wherein said apolipoprotein or fragment thereof is selected from the group consisting of ApoA1 (SEQ ID NO:402), ApoA2 (SEQ ID NO:403), ApoA4 (SEQ ID NO:404), ApoA5 (SEQ ID NO:405), ApoB48 (SEQ ID NO:406), ApoC1 (SEQ ID NO:407), ApoC2 (SEQ ID NO:408), ApoC3 (SEQ ID NO:409), ApoC4 (SEQ ID NO:410), ApoD (SEQ ID NO:411), ApoE2 (SEQ ID NO:283), ApoE3 (SEQ ID NO:284), ApoE4 (SEQ ID NO:285), ApoH (SEQ ID NO:412), ApoM (SEQ ID NO:413) and isoforms thereof.
16. Fusion polypeptide according to any one of items 1-15, wherein said apolipoprotein or fragment thereof is selected from ApoE and isoforms thereof and fragments thereof.
17. Fusion polypeptide according to any one of items 1-16, wherein said apolipoprotein or fragment thereof is selected from the group consisting of ApoE isoforms ApoE isoform 2 (SEQ ID NO:283), ApoE isoform 3 (SEQ ID NO:284) and ApoE isoform 4 (SEQ ID NO:285) and fragments thereof, such as wherein said apolipoprotein or fragment thereof is ApoE isoform 2 (SEQ ID NO:283) or a fragment thereof.
18. Fusion polypeptide according to any one of items 1-17, wherein said apolipoprotein or fragment thereof comprises a least one mutation, such as one, two, three, four or five or more mutations.
19. Fusion polypeptide according to items 18, wherein said at least one mutation, such as two, three, four or five mutations or more, reduce(s) the affinity of the apolipoprotein or fragment thereof for LDL-R and/or heparan sulphate proteoglycan, such as wherein said at least one mutation, such as two, three, four or five mutations or more, abolish(es) the affinity of the apolipoprotein or fragment thereof for LDL-R and/or heparan sulphate proteoglycan.
20. Fusion polypeptide according to any one of items 18-19, said at least one mutation is located in the heparan sulphate proteoglycan binding domain, such as wherein said at least one mutation is located in the region as defined by amino acid residues 144-147 as defined in SEQ ID NO:283 or in the corresponding region of a fragment thereof.
21. Fusion polypeptide according to any one of items 18-19, wherein said at least one mutation is located the LDL-R binding domain, such as wherein said at least one mutation is located in the region as defined by amino acid residues 140-150 as defined in SEQ ID NO:283 or in the corresponding region of a fragment thereof.
22. Fusion polypeptide according to any one of items 18-21, wherein said at least one mutation, such as two, three, four or five mutations or more, is/are located in a mutation site region, which region corresponds to the amino acid residues 132-158 ELRVRLASHLRKLRKRLLRDADDLQKC (SEQ ID NO:387) of SEQ ID NO:283 or in the corresponding region of a fragment thereof.
23. Fusion polypeptide according to any one of items 18-22, wherein said at least one mutation, such as two, three, four or five mutations, is/are selected from the group consisting of mutations in amino acid positions 136, 140, 143, 144 and 150 as defined in SEQ ID NO:283 or in the corresponding position(s) in a fragment thereof, such as wherein said at least one mutation, such as two, three, four or five mutations, is/are selected from the group consisting of R136S, H140A, K143A, L144P, and R150A, such as is/are selected from the group consisting of R136S, H140A, K143A, L144P, and R150A in amino acid position 136, 140, 143, 144 and 150, respectively, as defined in SEQ ID NO:283 or in the corresponding position in a fragment thereof.
24. Fusion polypeptide according to any one of items 1-23, wherein said apolipoprotein or fragment thereof comprises a mutation in amino acid position 61 as defined in SEQ ID NO:283 or in the corresponding region of a fragment thereof, such as wherein said apolipoprotein or fragment thereof comprises the mutation R61T as defined in SEQ ID NO:283 or in the corresponding region of a fragment thereof, such as in amino acid position 61 as defined in SEQID NO:283 or in the corresponding position in a fragment thereof.
25. Fusion polypeptide according to any one of items 1-24, wherein said apolipoprotein or fragment thereof is selected from the group consisting of SEQ ID NO: 389, 18, 391-401 and fragments thereof or wherein said apolipoprotein or fragment thereof is selected from the group consisting of SEQ ID NO:18 and 395-401 and fragments thereof, such as wherein said apolipoprotein or fragment thereof is selected from the group consisting of SEQ ID NO:18 and fragments thereof, which fragments comprise the R136S mutation.
26. Fusion polypeptide according to any one of items 1-19, wherein said apolipoprotein or fragment thereof does not comprise a heparan sulphate proteoglycan binding domain or comprises a heparan sulphate proteoglycan binding domain with reduced affinity for heparan sulphate proteoglycan compared to the wildtype domain, such as wherein said apolipoprotein or fragment thereof does not comprise a heparan sulphate proteoglycan binding domain.
27. Fusion polypeptide according to item26, wherein said apolipoprotein or fragment thereof does not comprise amino acid residues 144-147 as defined in SEQ ID NO:283.
28. Fusion polypeptide according to any one of items 1-19, wherein said apolipoprotein or fragment thereof does not comprise an LDL-R binding domain or comprises an LDL-R binding domain with reduced affinity for LDL-R compared to the wildtype domain, such as wherein said apolipoprotein or fragment thereof does not comprise an LDL-R binding domain.
29. Fusion polypeptide according to item 28, wherein said apolipoprotein or fragment thereof does not comprise amino acid residues 140-150 as defined in SEQ ID NO:283.
30. Fusion polypeptide according to any one of items 1-23, wherein said apolipoprotein or fragment thereof is SEQ ID NO:18 or a fragment thereof, which fragment comprises the R136S mutation.
31. Fusion polypeptide according to any one of items 1-30, wherein said tag moiety is fused, optionally via a linker, to an amino acid residue position corresponding to fusion site 1, 2 or 3 of SEQ ID NO:283, wherein fusion site 1 corresponds to the amino acid residues 1-12 KVEQAVETEPEP (SEQ ID NO:385), fusion site 2 corresponds to the amino acid residues 42-55 TLSEQVQEELLSSQ (SEQ ID NO:386), and fusion site 3 corresponds to the amino acid residues 162-201 YQAGAREGAERGLSAIRERLGPLVEQGRVRAATVGSLAGQ (SEQ ID NO:388) of ApoE isoform 2 (SEQ ID NO:283), and wherein all amino acid residues N-terminal to said amino acid residue position are not present.
32. Fusion polypeptide according to any one of items 1-31, wherein said apolipoprotein or fragment thereof fragment is selected from the group consisting of SEQ ID NO:19, SEQ ID NO:20, SEQ ID NO:21, SEQ ID NO:22 and SEQ ID NO:286.
33. Fusion polypeptide according to item 32, wherein said tag moiety as defined in any one of items 1-12 is fused, optionally via a linker,
   to apolipoprotein or fragment thereof having an amino acid sequence selected from the group consisting of SEQ ID NO:18, SEQ ID NO:19, SEQ ID NO:20, SEQ ID NO:21, SEQ ID NO:22 and SEQ ID NO:286, such as to a apolipoprotein fragment having an amino acid sequence selected from the group consisting of SEQ ID NO:19, SEQ ID NO:20, SEQ ID NO:21, SEQ ID NO:22 and SEQ ID NO:286.
34. Fusion polypeptide according to any one of items 1-33, wherein said fusion polypeptide is a fusion polypeptide comprising an amino acid sequence selected from the group consisting of SEQ ID NO:33-62, 74-103, 115-144, 156-185, 197-226, 297-326 and 331-384.
35. Fusion polypeptide according to any one of items 1-34, wherein said fusion polypeptide is a fusion polypeptide comprising an amino acid sequence selected from the group consisting of SEQ ID NO: 43-54, 84-93, 125-134, 166-175, 207-216, 307-316 and 349-366, such as the group consisting of SEQ ID NO:43, 84, 125, 166, 207 and 307 .
36. Fusion polypeptide according to any one of items 1-35, wherein said fusion polypeptide is a fusion polypeptide comprising an amino acid sequence selected from the group consisting of SEQ ID NO: 33, 43, 53, 74, 84, 94, 115, 125, 125, 156, 166, 176, 197, 207, 217, 297, 307 and 317.
37. A loaded decorated liponanoparticle comprising a core surrounded by a surface layer, wherein said core comprises at least one payload and said surface layer comprises:
   at least one phospholipid; and
   at least one fusion polypeptide as defined in any one of items 1-36.
38. Loaded decorated liponanoparticle according to item 37, further comprising at least one lipid, at least one triglyceride and/or at least one sterol, such as cholesterol.
39. Loaded decorated liponanoparticle according to any one of items 37-38, further comprising:
   (a) at least one cationic lipid;
   (b) at least one non-cationic lipid; and/or
   (c) at least one lipid conjugate,
   wherein said at least one cationic lipid, said at least one non-cationic lipid, and/or said at least one lipid conjugate is optionally chemically modified, such as chemically modified at its terminus.
40. Loaded decorated liponanoparticle according to any one of items 37-39,
   wherein said apolipoprotein or fragment thereof is bound to one component of the liponanoparticle.
41. Loaded decorated liponanoparticle according to item 40, wherein said apolipoprotein or fragment thereof is bound by hydrophobic bonds, hydrophilic bonds or non-covalent bonds.
42. Loaded decorated liponanoparticle according to item 40, wherein said apolipoprotein or fragment thereof is bound by covalent bonds.
43. Loaded decorated liponanoparticle according to any one of items 37-40 and 42, wherein said apolipoprotein or fragment thereof is covalently bound to said at least one cationic lipid, is covalently bound to said at least one non-cationic lipid, or is covalently bound to said at least one lipid conjugate; such as is covalently bound to the terminus of said at least one cationic lipid, such as is covalently bound to the terminus of said at least one non-cationic lipid, or such as is covalently bound to the terminus of said at least one lipid conjugate, wherein optionally said terminus is chemically modified.
44. Loaded decorated liponanoparticle according to any one of items 39-43, wherein said chemical modification is pegylation.
45. Loaded decorated liponanoparticle according to any one of items 37-44,
   wherein said payload exhibits a therapeutic and/or prophylactic activity upon delivery to a target cell.
46. Loaded decorated liponanoparticle according to any one of items 37-45,
   wherein said payload is a nucleic acid payload selected from the group consisting of RNA, DNA and nucleic acid analogs.
47. Loaded decorated liponanoparticle according to item 46, wherein said RNA is selected from the group consisting of microRNA (miRNA) , small interfering RNA (siRNA), piwi-interacting RNA (piRNA), small nuclear RNA (snoRNA), transfer RNA (tRNA), tRNA-derived small RNA (tsRNA), small regulatory RNA (srRNA), messenger RNA (mRNA), modified mRNA, ribosomal RNA (rRNA), long non-coding RNA (IncRNA) or guide RNA (gRNA) or combinations thereof and/or modifications thereof, such as wherein said RNA is messenger RNA (mRNA).
48. Loaded decorated liponanoparticle according to item 46, wherein said payload is antisense oligonucleotide, such as a single strand DNA or RNA oligo-nucleotide.
49. Loaded decorated liponanoparticle according to any one of items 46-48, wherein said payload is codon optimized and/or comprise modified nucleosides, such as naturally modified nucleotides and/or synthetic nucleoside analogues.
50. Loaded decorated liponanoparticle according to item 46, wherein said DNA is single stranded or double stranded DNA.
51. Loaded decorated liponanoparticle according to any one of items 46-50, wherein said nucleic acid is an antisense oligonucleotide and the antisense oligonucleotide is single strand DNA or RNA consisting of nucleotide or nucleoside analog containing modifications of the phosphodiester backbone or the 2' ribose.
52. Loaded decorated liponanoparticle according to item 52, wherein said nucleotide or nucleoside analog is selected from the group consisting of locked nucleic acid (LNA), bridged nucleic acid (BNA), morpholino or peptide nucleic acid (PNA).
53. A complex comprising a bispecific binding molecule and a loaded decorated liponanoparticle, wherein said bispecific binding molecule comprises:
   i) at least one first specific binding molecule with affinity for a target antigen;
   ii) at least one second specific binding molecule with affinity for a tag moiety as defined in any one of items1-12,

   wherein said first specific binding molecule and said second specific binding molecule are antigen-binding proteins each comprising an antigen-binding domain of an antibody and are covalently linked,
   wherein said loaded decorated liponanoparticle is as defined in any one of items 37-52 and is non-covalently bound to said second specific binding molecule via said tag moiety.
54. Complex according to item 53, wherein said first specific binding molecule has affinity for CD40.
55. Complex according to item 53 or 54, wherein said first specific binding molecule is an agonist of CD40.
56. Complex according to any one of items 53-55, wherein said first specific binding molecule induces CD40 internalization upon binding to CD40.
57. Complex according to any one of items 53-56, wherein said first specific binding molecule is an anti-CD40 antibody or antigen binding fragment thereof, such as an scFv.
58. Complex according to item 57, wherein said anti-CD40 antibody is selected from the group consisting of CP-870,893, APX005M, ADC-1013, ChiLob 7/4, SEA-CD40 and ABS-1150/1151 and an antibody comprising an antigen binding fragment derived from any one or more of said antibodies.
59. Complex according to item 57, wherein said anti-CD40 antibody or antigen binding fragment thereof comprises six complementarity determining domains (CDRs), wherein:
   VLCDR1 has the sequence set forth in SEQ ID NO:259;
   VLCDR2 has the sequence "AAS";
   VLCDR3 has the sequence set forth in SEQ ID NO:261;
   VHCDR1 has the sequence set forth in SEQ ID NO:262;
   VHCDR2 has the sequence set forth in SEQ ID NO:263; and
   VHCDR3 has the sequence set forth in SEQ ID NO:264.
60. Complex according to item 59, wherein the light chain variable domain of said anti-CD40 antibody comprises an amino acid sequence selected from SEQ ID NO:265 and amino acid sequences having at least 90 % sequence identity thereto; and the heavy chain variable domain of said anti-CD40 antibody comprises an amino acid sequence selected from SEQ ID NO:266 and amino acid sequences having at least 90 % sequence identity thereto.
61. Complex according to item 59 or 50, wherein said anti-CD40 antibody comprises
   a light chain comprising an amino acid sequence selected from SEQ ID NO:442 and amino acid sequences having at least 90 % sequence identity thereto; and
   a heavy chain comprising an amino acid sequence selected from SEQ ID NO:443 and amino acid sequences having at least 90 % sequence identity thereto.
62. Complex according to item 53, wherein said first specific binding molecule has affinity for HER2.
63. Complex according to item 51 or 60, wherein said first specific binding molecule is selected from the group consisting of trastuzumab, pertuzumab, margetuximab and antigen-binding fragments thereof, such as the group consisting of trastuzumab and antigen binding fragments thereof.
64. Complex according to any one of item 51 or 60-61, wherein said first specific binding molecule a light chain variable domain comprising an amino acid sequence selected from SEQ ID NO:268 and amino acid sequences having at least 90 % sequence identity thereto; and
   a heavy chain variable domain comprising an amino acid sequence selected from SEQ ID NO:267 and amino acid sequences having at least 90 % sequence identity thereto.
65. Complex according to item 51, wherein said first specific binding molecule has affinity for CD20.
66. Complex according to item 51 or 63, wherein said first specific binding molecule is selected from the group consisting of rituximab, ocrelizumab, obinutuzumab, ofatumumab, ibritumomab, tiuxetan, tositumomab, and ublituximab and antigen binding fragments thereof, such as the group consisting of rituximab and antigen binding fragments thereof
67. Complex according to any one of item 51 or 63-64, wherein said first specific binding molecule a light chain variable domain comprising an amino acid sequence selected from SEQ ID NO:270 and amino acid sequences having at least 90 % sequence identity thereto; and
   a heavy chain variable domain comprising an amino acid sequence selected from SEQ ID NO:269 and amino acid sequences having at least 90 % sequence identity thereto.
68. Complex according to any one of items 53-67, wherein said second specific binding molecule is an antibody or an antigen binding fragment thereof.
69. Complex according to any one of items 53-67, wherein said second specific binding molecule comprises:
   an immunoglobulin heavy chain variable region (VH) comprising three complementarity determining domains (CDRs), wherein:
      VHCDR1 has the sequence set forth in SEQ ID NO:252;
      VHCDR2 has the sequence IGRIDPEXaXbDAEYVP (SEQ ID NO:253), wherein
      XaXb is selected from the group consisting of SG, GG, QG, DG, NA and NG and;
      VHCDR3 has the sequence set forth in SEQ ID NO:254; and
   an immunoglobulin light chain variable region (VL) comprising three complementarity determining domains (CDRs), wherein:
      VLCDR1 has the sequence set forth in SEQ ID NO:256;
      VLCDR2 has the sequence set forth in SEQ ID NO:257; and
      VLCDR3 has the sequence set forth in SEQ ID NO:257.
70. Complex according to item 69, wherein said second specific binding molecule comprises
   - an immunoglobulin heavy chain variable region (VH) consisting of an amino acid sequence selected from the group consisting of SEQ ID NO: 246-251 and amino acid sequences having at least 90 % identity thereto; and
   - an immunoglobulin light chain variable region (VL) consisting of an amino acid sequence set forth in SEQ ID NO:255 and amino acid sequences having at least 90 % identity thereto.
71. Complex according to any one of items 51-66, wherein said second specific binding molecule is an scFv.
72. Complex according to item 70, wherein said second specific binding molecule comprises a complete scFv sequence selected from the group consisting of SEQ ID NO: 436-441, for example selected from the group consisting of SEQ ID NO:436 and 441, for example SEQ ID NO:436.
73. Complex according to item 71, wherein said second specific binding molecule comprises the complete scFv sequence SEQ ID NO:436.
74. Complex according to any one of items 53-61 and 68-73, comprising a bispecific binding molecule and a loaded decorated liponanoparticle, wherein said bispecific binding molecule consists of two copies of a polypeptide chain comprising SEQ ID NO:446, and two copies of a polypeptide chain comprising SEQ ID NO:442.
75. Complex according to any one of items 53-74, wherein said second specific binding molecule is capable of biding to said tag moiety as defined in any one of items 1-12 such that the K_{D} value of the interaction is at most 1 x 10⁻⁹ M, for example at most 1 x 10⁻¹⁰ M, for example at most 1 x 10⁻¹¹ M.
76. A polynucleotide encoding a fusion polypeptide according to any one of items 1-36.
77. An expression vector comprising a polynucleotide according to item 76.
78. A host cell comprising an expression vector according to item 77.
79. A composition comprising a fusion polypeptide according to any one of items 1-36 and at least one pharmaceutically acceptable excipient or carrier.
80. A composition comprising a loaded decorated liponanoparticle according to any one of items 37-52 or a complex according to any one of items 53-75 and at least one pharmaceutically acceptable excipient or carrier.
81. A kit comprising a loaded decorated liponanoparticle according to any one of items 37-52, and at least one bispecific binding molecule as defined in item 53-75; and instructions for use of said kit.
82. A method of producing a fusion polypeptide according to any one of items 1-36, the method comprising:
   - culturing a host cell according to item 78 under conditions allowing expression of said fusion polypeptide from said expression vector; and
   - isolating said fusion polypeptide.
83. A method of producing a fusion polypeptide according to any one of items 1-36, the method comprising:
   - step-wise coupling of the amino acids and/or the amino acid derivatives to form a polypeptide according to the first aspect having protected reactive side-chains,
   - removal of the protecting groups from the reactive side-chains of the polypeptide; and thereby
   - obtaining of the polypeptide in aqueous solution.
84. A method of producing a loaded decorated liponanoparticle as defined in any one of items 37-52, the method comprising:
   - combining at least one phospholipid, and optionally at least one sterol and/or at least one lipid, with a payload, such as a payload as defined in any one of items 45-52, to obtain a loaded liponanoparticle;
   - providing at least one fusion polypeptide according to any one of items 1-36; and
   - combining said at least one fusion polypeptide with phospholipids, and optionally sterols and/or lipids, to obtain said loaded decorated liponanoparticle.
85. A method of producing a loaded decorated liponanoparticle as defined in any one of items 37-52, the method comprising:
   - combining at least one ionizable lipid; 1,2-distearoyl-sn-glycero-3-phosphocholine; cholesterol; at least one stabilizer, such as 1,2-Dimyristoyl-rac-glycero-3-methoxypolyethylene glycol-2000, with a payload, such as a payload as defined in any one of items 45-52, to obtain a loaded liponanoparticle;
   - providing at least one fusion polypeptide according to any one of items 1-36; and
   - combining said at least one fusion polypeptide with phospholipids, and optionally sterols and/or lipids, to obtain said loaded decorated liponanoparticle.
86. A method of producing a complex as defined in any one of items 53-75, the method comprising:
   - providing at least one bispecific binding molecule as defined in any one of items 53-75;
   - providing at least one loaded decorated liponanoparticle according to any one of items 37-52; and
   - combining said at least one bispecific binding molecule with said at least one loaded decorated liponanoparticle, to obtain said complex.
87. A fusion polypeptide according to any one of items 1-36, a loaded decorated liponanoparticle according to any one of items 37-52, complex according to any one of items 53-75 or a kit according to item 81 for use in therapy of a subject in need thereof.
88. A product comprising a bispecific binding molecule as defined in any one of items 53-75 and a loaded decorated liponanoparticle according to any one of items 37-52 as a combined preparation for separate, simultaneous or sequential use in therapy of a subject in need thereof.
89. A fusion polypeptide according to any one of items 1-36, a loaded decorated liponanoparticle according to any one of items 37-52, a complex according to any one of items 53-75, a composition according to any one of items 79-80, or a kit according to item 81 for use as a diagnostic agent *in vitro,* for use as a diagnostic agent *in vivo,* for use as a prognostic agent *in vitro,* and/or for use as a prognostic agent *in vivo.*
90. A method of treatment of a disease, disorder or infection, comprising administering to a subject in need thereof of a therapeutically effective amount of a fusion polypeptide according to any one of items 1-36, a loaded decorated liponanoparticle according to any one of items 37-52, a complex according to any one of items 53-75, or a composition according to any one of items 79-80.
91. Use of a fusion polypeptide according to any one of items 1-36, a loaded decorated liponanoparticle according to any one of items 37-52, a complex according to any one of items 53-75, a composition according to any one of items 79-80, or a kit according to item 81 in the manufacture of a medicament.
92. A fusion polypeptide according to any one of items 1-36, a loaded decorated liponanoparticle according to any one of items 37-52, complex according to any one of items 53-75 or a kit according to item 81 for use as a diagnostic agent in the *in vitro* or *in vivo* diagnosis of a disease, disorder and/or infection.
93. A fusion polypeptide according to any one of items 1-36, a loaded decorated liponanoparticle according to any one of items 37-52, complex according to any one of items 53-75 or a kit according to item 81 for use as a prognostic agent in the *in vitro* or *in vivo* prognosis of a disease, disorder and/or infection.
94. Method for determining the presence of a marker, such as a marker indicating a disease, disorder and/or infection, in a subject or in a sample isolated from said subject, comprising the steps of:
   a) contacting said subject or said sample isolated from said subject, with a loaded decorated liponanoparticle according to any one of items 37-52, a complex according to any one of items 53-75, a composition according to any one of items 79-80; and
   b) obtaining a value corresponding to the amount of loaded decorated liponanoparticle, complex or composition that has bound in said subject or to said sample.
95. Method of diagnosis or prognosis, comprising the steps of:
   a) contacting a subject or a sample isolated from said subject, with a loaded decorated liponanoparticle according to any one of items 37-52, a complex according to any one of items 53-75, a composition according to any one of items 79-80; and
   b) obtaining a value corresponding to the amount of loaded decorated liponanoparticle, complex or composition that has bound in said subject or to said sample.
96. Method of diagnosis or prognosis according to item 95, which is a method for diagnosis *in vivo* or a method for prognosis *in vivo,* for example via medical imaging, in which said contacting in step a) consists of contacting said subject with said loaded decorated liponanoparticle, complex or composition.
97. Method of diagnosis or prognosis according to item 94, which is a method for diagnosis *in vitro* or method for prognosis *in vitro,* in which said contacting in step a) consists of contacting a sample previously isolated from said subject with said loaded decorated liponanoparticle, complex or composition.
98. Method of diagnosis or prognosis according to any one of items 95-97,
   wherein said subject is a mammalian subject, such as a human subject.
99. An *in vivo* or *in vitro* method for introducing a payload, such as a nucleic acid or a nucleic acid analog, such as RNA, DNA or nucleic acid analog as defined in any one of items 45-52, into at least one cell, the method comprising:
   - contacting the loaded decorated liponanoparticle according to any one of items 37-52 or the composition according to any one of items 79-80, with said at least one cell.
100. An *in vivo* or *in vitro* method for introducing a payload, such as a nucleic acid or a nucleic acid analog, such as RNA, DNA or nucleic acid analog as defined in any one of items 45-52, into at least one cell, the method comprising:
   - contacting the complex comprising a bispecific binding molecule and a loaded decorated liponanoparticle according to any one of items 53-75 with said at least one cell, wherein said at least one cell expresses the target antigen for which said at least one first specific binding molecule of said bispecific binding molecule has affinity.

## Claims

1. Fusion polypeptide comprising
iii) a tag moiety comprising or consisting of the amino acid sequence FIGITELX₈X₉ (SEQ ID NO:1)
wherein
X₈ is selected from K, L, and H; and
X₉ is K or absent; and
iv) a polypeptide comprising an apolipoprotein or a fragment thereof,
said tag moiety is arranged N-terminally of said apolipoprotein or fragment thereof and wherein said fusion polypeptide optionally comprises a linker sequence arranged between said tag moiety and said apolipoprotein or fragment thereof.

2. Fusion polypeptide according to claim 1, wherein said tag moiety comprises or consists of an amino acid sequence selected form the group consisting of SEQ ID NO:2-4.

3. Fusion polypeptide according to claim 1 or 2, wherein said tag moiety comprises or consists of the amino acid sequence as defined in SEQ ID NO:3.

4. Fusion polypeptide according to any one of claims 1-3, wherein said apolipoprotein is selected from the group consisting of ApoE isoforms ApoE isoform 2 (SEQ ID NO:283), ApoE isoform 3 (SEQ ID NO:284) and ApoE isoform 4 (SEQ ID NO:285) and fragments thereof, such as wherein said apolipoprotein is ApoE isoform 2 (SEQ ID NO:283) or a fragment thereof.

5. Fusion polypeptide according to any one of claims 1-4, wherein said apolipoprotein or fragment thereof comprises a least one mutation, such as one, two, three, four or five or more mutations, which reduce(s) the affinity of the apolipoprotein or fragment thereof for LDL-R and/or heparan sulphate proteoglycan, such as wherein said at least one mutation, such as two, three, four or five mutations, abolish(es) the affinity of the apolipoprotein or fragment thereof for LDL-R and/or heparan sulphate proteoglycan.

6. Fusion polypeptide according to claim 5, wherein said at least one mutation, such as two, three, four or five mutations or more, is/are selected from the group consisting of R136S, H140A, K143A, L144P, and R150A as defined in SEQ ID NO:283 or the corresponding mutation in a fragment thereof.

7. Fusion polypeptide according to claim 6, wherein said tag moiety as defined in any one claims 1-3 is fused, optionally via a linker,
to apolipoprotein or fragment thereof having an amino acid sequence selected from the group consisting of SEQ ID NO:18, SEQ ID NO:19, SEQ ID NO:20, SEQ ID NO:21, SEQ ID NO:22 and SEQ ID NO:286, such as to a apolipoprotein fragment having an amino acid sequence selected from the group consisting of SEQ ID NO:19, SEQ ID NO:20, SEQ ID NO:21, SEQ ID NO:22 and SEQ ID NO:286.

8. A loaded decorated liponanoparticle comprising a core surrounded by a surface layer, wherein said core comprises at least one payload and said surface layer comprises:
at least one phospholipid; and
at least one fusion polypeptide as defined in any one of claims 1-7.

9. Loaded decorated liponanoparticle according to claim 8, wherein said payload is a nucleic acid payload selected from the group consisting of RNA, DNA and nucleic acid analogs.

10. A complex comprising a bispecific binding molecule and a loaded decorated liponanoparticle, wherein said bispecific binding molecule comprises:
iii) at least one first specific binding molecule with affinity for a target antigen, such as a peptide antigen;
iv) at least one second specific binding molecule with affinity for a tag moiety as defined in any one of claims 1-3,
wherein said first specific binding molecule and said second specific binding molecule are antigen-binding proteins each comprising an antigen-binding domain of an antibody and are covalently linked,
wherein said loaded decorated liponanoparticle is as defined in any one of claims 8-9 and is non-covalently bound to said second specific binding molecule via said tag moiety.

11. Complex according to claim 10, wherein said first specific binding molecule is an anti-CD40 antibody which comprises
a light chain variable domain comprising an amino acid sequence selected from SEQ ID NO:265 and amino acid sequences having at least 90 % sequence identity thereto; and
a heavy chain variable domain comprising an amino acid sequence selected from SEQ ID NO:266 and amino acid sequences having at least 90 % sequence identity thereto.

12. Complex according to any one of claims 10-11, wherein said second specific binding molecule comprises the complete scFv sequence selected from the group consisting of SEQ ID NO:436-441, such as wherein said second specific binding molecule comprises the complete scFv sequence SEQ ID NO:436.

13. Complex according to any one of claims 10-12, comprising a bispecific binding molecule and a loaded decorated liponanoparticle, wherein said bispecific binding molecule consists of two copies of a polypeptide chain comprising SEQ ID NO:446, and two copies of a polypeptide chain comprising SEQ ID NO:442.

14. A fusion polypeptide according to any one of claims 1-7, a loaded decorated liponanoparticle according to any one of claims 8-9, complex according to any one of claims 10-13 for use in therapy of a subject in need thereof.

15. A product comprising a bispecific binding molecule as defined in any one of claims 10-13 and loaded decorated liponanoparticle according to any one of claims 8-9 as a combined preparation for separate, simultaneous or sequential use in therapy of a subject in need thereof.
